# EUROPEAN PATENT APPLICATION

(11) **EP 4 610 257 A1**
(43) Date of publication of application: **03.09.2025**
(21) Application number: 23881977.5
(22) Date of filing: 27.10.2023
(51) Int. Cl.: C07D 471/04, A61P 11/08, A61K 31/519, A61P 37/08, A61P 11/06

(54) **CRYSTAL FORM OF ISOQUINOLINONE COMPOUND AND PREPARATION METHOD THEREFOR**

(30) Priority: 28.10.2022 CN 202211336045
(71) Applicant: Jiangsu Hengrui Pharmaceuticals Co., Ltd., Lianyungang, Jiangsu 222047 (CN)
(72) Inventor: SHANG, Tingting, Lianyungang, Jiangsu 222047 (CN); YANG, Junran, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: dompatent von Kreisler Selting Werner - Partnerschaft von Patent- und Rechtsanwälten mbB
(86) International application number: PCT/CN2023/127230
(87) International publication number: WO 2024/088402

(57) **Abstract**

The present disclosure relates to a crystal form of an isoquinolinone compound and a preparation method therefor. Specifically, provided in the present disclosure are a crystal form of a compound as represented by formula (I) and a preparation method therefor.

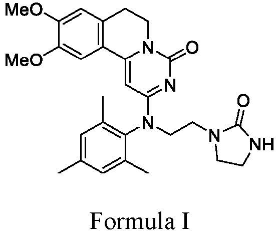

## Description

The present application claims priority to Chinese Patent Application No. 2022113360457 filed on October 28, 2022, which is incorporated herein by reference in its entirety.

### TECHNICAL FIELD

The present disclosure relates to crystal forms of an isoquinolinone compound and preparation methods therefor and particularly provides crystal forms of the compound represented by formula I and preparation methods therefor.

### BACKGROUND

Phosphodiesterases (PDEs) belong to a superfamily of enzyme systems including 11 families, each of which plays a role in a different signal transduction and regulates a different physiological process. Among them, PDE3 has the ability to hydrolyze both cAMP and cGMP, and its hydrolysis ability for cAMP is about ten times greater than that for cGMP. There are two genetic subtypes of PDE3: PDE3A and PDE3B, which are located on chromosome 11 and chromosome 12, respectively. According to the start codon, PDE3A can be subdivided into three subtypes: PDE3A1, PDE3A2, and PDE3A3, which are present mainly in the heart, platelets, the vascular smooth muscle, and oocytes, serving to regulate myocardial contractility, platelet aggregation, vascular smooth muscle contraction, oocyte maturation, renin release, etc. There is only one subtype of PDE3B: PDE3B1, which is mainly present in lipocytes, liver cells, spermatocytes, and the pancreas and is mainly involved in the regulation of the signal transduction of insulin, insulin-like growth factors, and leptin. It plays an important role in metabolic diseases such as obesity and diabetes. Main PDE3 selective inhibitors include cilostazol, cilostamide, milrinone, amrinone, enoximone, siguazodan, etc.

For example, amrinone inhibits PDE3 activity, increases the cAMP concentration in myocardial cells, and increases the Ca²⁺ concentration in cells, thereby producing a positive inotropic effect to the full. Meanwhile, amrinone directly acts on vascular smooth muscle cells, producing a good vasodilating effect, increasing myocardial contractility, reducing pulmonary artery pressure, and restoring heart and lung function. It has great value in the treatment of chronic pulmonary heart disease combined with heart failure. In addition, cilostazol is clinically used for treating antiplatelet aggregation, pulmonary hypertension (PAH), chronic obstructive pulmonary disease (COPD), intermittent claudication, and cerebral small vessel disease.

In another aspect, PDE4 is highly specific for cAMP, and there are 4 subtypes of PDE4: PDE4A, PDE4B, PDE4C, and PDE4D. PDE4 is involved in the promotion of monocyte and macrophage activation, neutrophil infiltration, vascular smooth muscle proliferation, vasodilatation, myocardial contraction, and other related pathophysiological processes, and has effects on central nervous system function, cardiovascular function, inflammation/the immune system, cell adhesion, etc. PDE4 plays a leading regulatory role in the expression of pro- and anti-inflammatory mediators. PDE4 inhibitors can inhibit the release of harmful mediators from inflammatory cells.

A new molecule with both PDE3 and PDE4 inhibitory activities can cause bronchodilation like a β-adrenergic receptor agonist and anti-inflammatory action like an inhaled glucocorticoid. The two complementary targeting abilities can achieve a better effect than a single one.

For example, RPL554 (9,10-dimethoxy-2-(2,4,6-trimethylphenylimino)-3-(*N*-carbamoyl-2 -aminoethyl)-3,4,6,7-tetrahydro-2*H*-pyrimido[6,1-*a*]isoquinolin-4-one) is a PDE3/PDE4 dual-target inhibitor disclosed in WO00/58308. Recent phase II clinical data show that the drug can significantly improve bronchodilation and symptoms in patients with chronic obstructive pulmonary disease, and that the drug was well tolerated and did not cause noticeable adverse events; for example, problems with the heart, nausea, and diarrhea were all mild. The safety of the drug and its "limited systemic exposure" are encouraging.

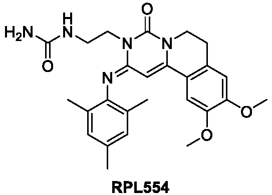

A new molecule with both PDE3 and PDE4 inhibitory activities can cause bronchodilation like a β-adrenergic receptor agonist and anti-inflammatory action like an inhaled glucocorticoid. The two complementary targeting abilities can achieve a better effect than a single one.

The patent application PCT/CN2022/090175 provides the compound represented by formula I, which has the chemical name 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]isoquinolin-4-one, and the compound has good inhibitory activity against PDE3 and PDE4:

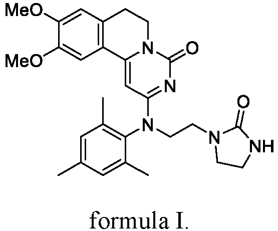

Generally, the crystalline structure of a drug's active ingredient and its salts has an impact not only on the drug's physical and chemical stability but also on the level of difficulty of the drug's late-stage preparation and production costs. Variations in crystallization and storage conditions can lead to changes in the crystalline structure of the compound and its salts and sometimes to the generation of other crystal forms. Therefore, it is necessary to conduct in-depth research on the crystal forms of the compound represented by formula I in view of stability, the level of difficulty of drug preparation processes, production cost, etc.

### SUMMARY

The present disclosure provides crystal forms of the compound represented by formula (I) and preparation methods therefor, wherein the compound represented by formula I has the chemical name 9,10-dimethoxy-2-[[2-(2-oxo-imidazolidin-1-yl)-ethyl]-(2,4,6-trimethyl-phenyl)-amino]-6,7-dihydro-pyrimido[6,1-*a*]isoquinolin-4-one:

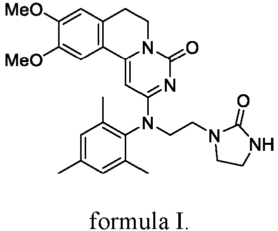

The present disclosure provides an amorphous form of the compound represented by formula (I), and an X-ray powder diffraction pattern of the amorphous form has no significant characteristic peaks within a 2θ diffraction angle range of 2° to 45°.

The present disclosure further provides a method for preparing the amorphous form of the compound represented by formula (I), the method comprising steps of mixing the compound represented by formula (I) with benzyl alcohol and dissolving, and b) crystallizing by volatilization.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I. In certain embodiments, the method for preparing the amorphous form of the compound represented by (I) of the present disclosure further comprises a filtration step, a washing step, or a drying step.

The present disclosure provides a crystal form A of the compound represented by formula (I), and an X-ray powder diffraction pattern expressed in terms of 2θ angles, which are diffraction angles, of the crystal form has characteristic peaks at 6.6, 11.0, 14.4, 15.5, 17.9, 19.1, and 23.7. In certain embodiments, the crystal form A of the compound represented by formula (I) has characteristic peaks at 4.6, 6.6, 10.1, 11.0, 14.4, 15.5, 17.9, 19.1, 22.8, and 23.7.

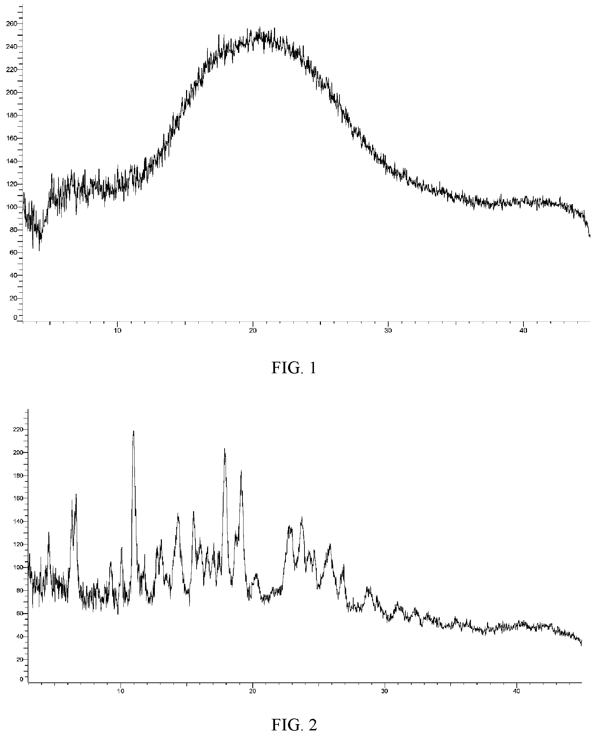

In certain embodiments, the crystal form A of the compound represented by formula (I) has characteristic peaks at 4.6, 6.6, 10.1, 11.0, 13.1, 14.4, 15.5, 17.1, 17.9, 19.1, 22.8, 23.7, and 25.9. In certain embodiments, the X-ray powder diffraction pattern expressed in terms of 2θ angles, which are diffraction angles, of the crystal form A of the compound represented by formula (I) is shown in FIG. 2.

The present disclosure provides a crystal form B of the compound represented by formula (I), and an X-ray powder diffraction pattern expressed in terms of 2θ angles, which are diffraction angles, of the crystal form has characteristic peaks at 6.6, 11.0, 12.7, 15.5, 19.1, 23.7, and 25.8. In certain embodiments, the crystal form B of the compound represented by formula (I) has characteristic peaks at 6.6, 10.0, 11.0, 11.7, 12.7, 15.5, 19.1, 23.7, 24.6, and 25.8.

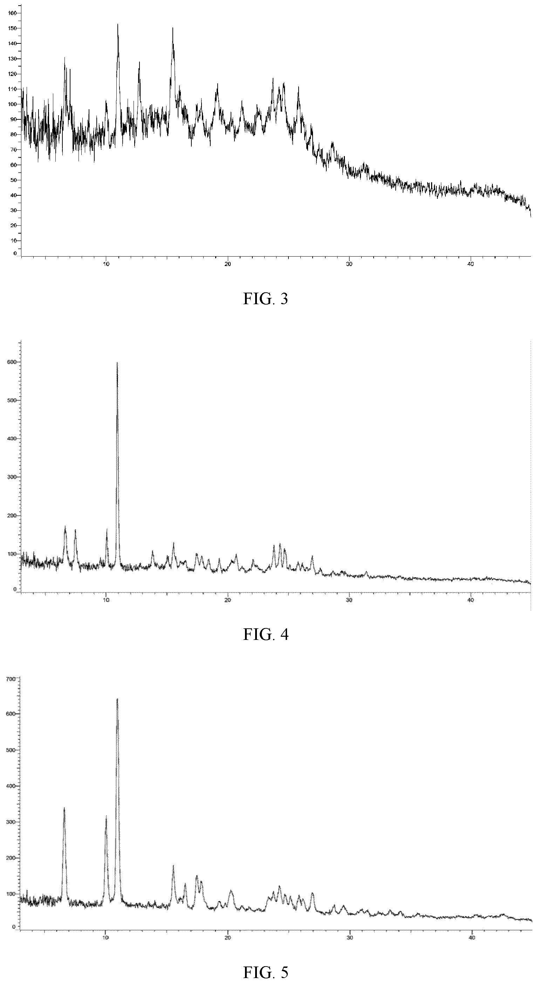

In certain embodiments, the crystal form B of the compound represented by formula (I) has characteristic peaks at 6.6, 8.6, 10.0, 11.0, 11.7, 12.7, 15.5, 17.8, 19.1, 23.7, 24.6, 25.8, and 26.9. In certain embodiments, the X-ray powder diffraction pattern expressed in terms of 2θ angles, which are diffraction angles, of the crystal form B of the compound represented by formula (I) is shown in FIG. 3.

The present disclosure further provides a method for preparing the crystal form B of the compound represented by formula (I), the method comprising: a) mixing the compound represented by formula I with dichloromethane and dissolving, and b) crystallizing by volatilization.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I. In certain embodiments, the method for preparing the crystal form B of the compound represented by formula (I) of the present disclosure further comprises a filtration step, a washing step, a drying step, or the like.

The present disclosure provides a crystal form C of the compound represented by formula (I), and an X-ray powder diffraction pattern expressed in terms of 2θ angles, which are diffraction angles, of the crystal form has characteristic peaks at 6.6, 7.5, 10.1, 10.9, 15.6, 23.8, and 24.3. In certain embodiments, the crystal form C of the compound represented by formula (I) has characteristic peaks at 6.6, 7.5, 10.1, 10.9, 13.8, 15.6, 20.7, 23.8, 24.3, and 24.7.

In certain embodiments, the crystal form C of the compound represented by formula (I) has characteristic peaks at 6.6, 7.5, 10.1, 10.9, 13.8, 15.6, 17.5, 19.3, 20.7, 23.8, 24.3, 24.7, and 27.0. In certain embodiments, the X-ray powder diffraction pattern expressed in terms of 2θ angles, which are diffraction angles, of the crystal form C of the compound represented by formula (I) is shown in FIG. 4.

The present disclosure further provides a method for preparing the crystal form C of the compound represented by formula (I), the method comprising: a) mixing the compound represented by formula I with acetonitrile, methanol, or a mixture thereof, and b) crystallizing by volatilization.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I. In certain embodiments, the method for preparing the crystal form C of the compound represented by formula (I) of the present disclosure further comprises a filtration step, a washing step, a drying step, or the like.

The present disclosure provides a crystal form D of the compound represented by formula (I), and an X-ray powder diffraction pattern expressed in terms of 2θ angles, which are diffraction angles, of the crystal form has characteristic peaks at 6.6, 10.1, 11.0, 15.6, 16.5, 17.5, and 24.2.

In certain embodiments, the crystal form D of the compound represented by formula (I) has characteristic peaks at 6.6, 10.1, 11.0, 15.6, 16.5, 17.5, 20.3, 24.2, 25.8, and 26.9.

In certain embodiments, the crystal form D of the compound represented by formula (I) has characteristic peaks at 6.6, 10.1, 11.0, 15.6, 16.5, 17.5, 20.3, 23.3, 24.2, 24.7, 25.1, 25.8, and 26.9.

In certain embodiments, the X-ray powder diffraction pattern expressed in terms of 2θ angles, which are diffraction angles, of the crystal form D of the compound represented by formula (I) is shown in FIG. 5.

The present disclosure further provides a method for preparing the crystal form D of the compound represented by formula (I), the method comprising:
method 1: a) mixing the compound represented by formula I with a solvent II, and dissolving with heating or without heating, wherein the solvent II is selected from the group consisting of one or more of propylene glycol methyl ether, 1,2-dichloroethane, ethyl acetate/ethanol, *n-*propanol, tetrahydrofuran/ethanol, trichloromethane, *N,N*-dimethylformamide, *N,N-*dimethylacetamide, ethanol/acetone, ethanol/isopropyl acetate, and acetonitrile/methanol/acetone, and b) crystallizing by volatilization;
or method 2: a) mixing the compound represented by formula I with a solvent III, and dissolving, wherein the solvent III is selected from the group consisting of one or more of ethanol, *N-*methylpyrrolidone, dichloromethane, and acetonitrile/methanol, and b) adding a solvent IV, and crystallizing, wherein the solvent IV is selected from the group consisting of one or more of methyl *tert*-butyl ether, *n*-heptane, water, acetone, and isopropyl acetate;
or method 3: a) mixing the compound represented by formula I with a solvent V, wherein the solvent V is selected from the group consisting of one or more of water, acetone, ethyl acetate, isopropyl acetate, methyl *tert*-butyl ether, 2-butanone, tetrahydrofuran, methyl isobutyl ketone, 1,4-dioxane, isoamyl alcohol, water/ethanol, water/isopropanol, water/acetone, methanol/water, ethyl acetate/*n*-heptane, *o*-xylene, isopropyl ether, and toluene, and b) crystallizing by stirring.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form D of the compound represented by formula (I) of the present disclosure further comprises a filtration step, a washing step, a drying step, or the like.

The present disclosure provides a crystal form E of the compound represented by formula (I), and an X-ray powder diffraction pattern expressed in terms of 2θ angles, which are diffraction angles, of the crystal form has characteristic peaks at 6.3, 11.1, 12.8, 14.3, 17.9, 22.9, and 25.6. In certain embodiments, the crystal form E of the compound represented by formula (I) has characteristic peaks at 6.3, 11.1, 12.8, 14.3, 17.0, 17.9, 18.7, 22.9, 23.8, and 25.6.

In certain embodiments, the crystal form E of the compound represented by formula (I) has characteristic peaks at 6.3, 11.1, 12.8, 14.3, 15.9, 17.0, 17.9, 18.7, 20.1, 22.9, 23.8, and 25.6.

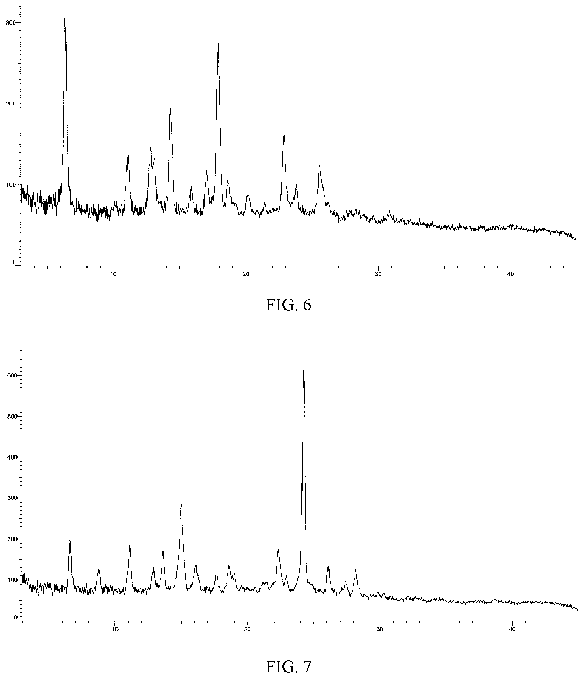

In certain embodiments, the X-ray powder diffraction pattern expressed in terms of 2θ angles, which are diffraction angles, of the crystal form E of the compound represented by formula (I) is shown in FIG. 6.

The present disclosure further provides a method for preparing the crystal form E of the compound represented by formula (I), the method comprising: a) mixing the compound represented by formula I with N-methylpyrrolidone and dissolving, and b) crystallizing by adding isopropyl acetate.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form E of the compound represented by formula (I) of the present disclosure further comprises a filtration step, a washing step, a drying step, or the like.

The present disclosure provides a crystal form F of the compound represented by formula (I), and an X-ray powder diffraction pattern expressed in terms of 2θ angles, which are diffraction angles, of the crystal form has characteristic peaks at 6.6, 11.1, 13.6, 15.0, 22.3, 24.2, and 26.1. In certain embodiments, the crystal form F of the compound represented by formula (I) has characteristic peaks at 6.6, 11.1, 13.6, 15.0, 16.1, 18.6, 22.3, 24.2, 26.1, and 28.2.

In certain embodiments, the crystal form F of the compound represented by formula (I) has characteristic peaks at 6.6, 8.8, 11.1, 12.9, 13.6, 15.0, 16.1, 17.7, 18.6, 22.3, 24.2, 26.1, and 28.2. In certain embodiments, the X-ray powder diffraction pattern expressed in terms of 2θ angles, which are diffraction angles, of the crystal form F of the compound represented by formula (I) is shown in FIG. 7.

The present disclosure further provides a method for preparing the crystal form F of the compound represented by formula (I), the method comprising:
method 1: a) mixing the compound represented by formula I with N-methylpyrrolidone and dissolving, and b) crystallizing by stirring;
or method 2: a) mixing the compound represented by formula I with N-methylpyrrolidone and dissolving, and b) crystallizing by adding methyl tert-butyl ether and n-heptane.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form F of the compound represented by formula (I) of the present disclosure further comprises a filtration step, a washing step, a drying step, or the like.

The present disclosure provides a crystal form G of the compound represented by formula (I), and an X-ray powder diffraction pattern expressed in terms of 2θ angles, which are diffraction angles, of the crystal form has characteristic peaks at 6.1, 12.3, 14.2, 16.9, 19.2, 21.4, and 24.0. In certain embodiments, the crystal form G of the compound represented by formula (I) has characteristic peaks at 6.1, 12.3, 13.1, 14.2, 15.2, 16.1, 16.9, 19.2, 21.4, and 24.0.

In certain embodiments, the crystal form G of the compound represented by formula (I) has characteristic peaks at 6.1, 11.3, 12.3, 13.1, 14.2, 15.2, 16.1, 16.9, 19.2, 21.4, 24.0, 26.6, and 28.8.

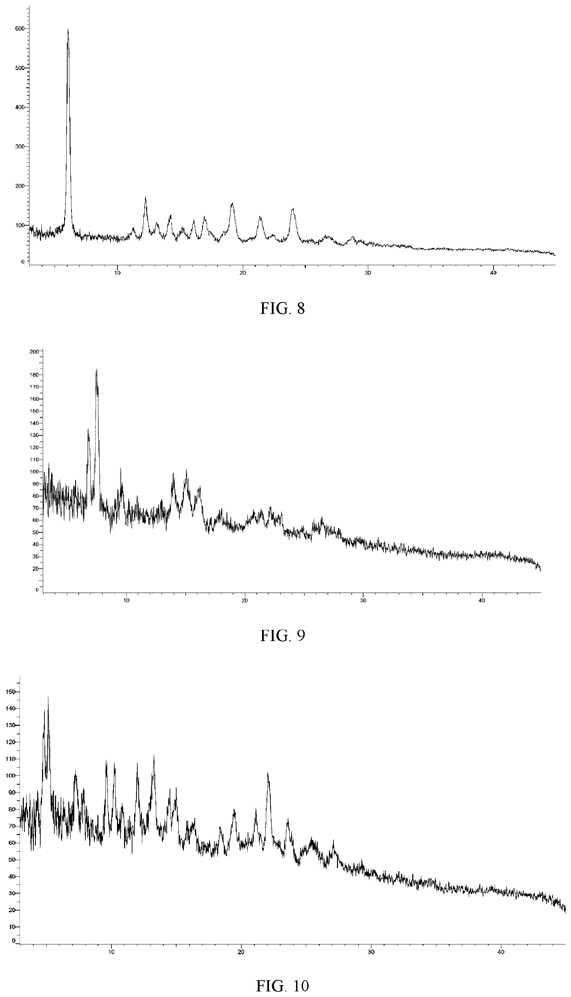

In certain embodiments, the X-ray powder diffraction pattern expressed in terms of 2θ angles, which are diffraction angles, of the crystal form G of the compound represented by formula (I) is shown in FIG. 8.

The present disclosure further provides a method for preparing the crystal form G of the compound represented by formula (I), the method comprising: a) mixing the compound represented by formula I with dichloromethane and dissolving, and b) crystallizing by adding isopropyl acetate or methyl tert-butyl ether.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form G of the compound represented by formula (I) of the present disclosure further comprises a filtration step, a washing step, a drying step, or the like.

The present disclosure provides a crystal form H of the compound represented by formula (I), and an X-ray powder diffraction pattern expressed in terms of 2θ angles, which are diffraction angles, of the crystal form has characteristic peaks at 6.8, 7.5, 9.7, 13.0, 14.0, 15.1, and 16.1.

In certain embodiments, the crystal form H of the compound represented by formula (I) has characteristic peaks at 6.8, 7.5, 9.7, 10.2, 10.9, 13.0, 14.0, 15.1, 16.1, and 26.5.

In certain embodiments, the crystal form H of the compound represented by formula (I) has characteristic peaks at 6.8, 7.5, 9.7, 10.2, 10.9, 13.0, 14.0, 15.1, 16.1, 22.1, 23.1, 25.8, and 26.5. In certain embodiments, the X-ray powder diffraction pattern expressed in terms of 2θ angles, which are diffraction angles, of the crystal form H of the compound represented by formula (I) is shown in FIG. 9.

The present disclosure further provides a method for preparing the crystal form H of the compound represented by formula (I), the method comprising:
method 1: a) mixing the crystal form A of the compound represented by formula I with methanol and heating, and b) crystallizing by cooling;
method 2: a) mixing the compound represented by formula I with an acetonitrile/methanol mixture and dissolving, and b) crystallizing by adding methyl tert-butyl ether.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I. In certain embodiments, the method for preparing the crystal form H of the compound represented by formula (I) of the present disclosure further comprises a filtration step, a washing step, a drying step, or the like.

The present disclosure provides a crystal form I of the compound represented by formula (I), and an X-ray powder diffraction pattern expressed in terms of 2θ angles, which are diffraction angles, of the crystal form has characteristic peaks at 5.2, 7.2, 9.6, 10.2, 12.0, 13.3, and 22.1.

In certain embodiments, the crystal form I of the compound represented by formula (I) has characteristic peaks at 5.2, 7.2, 9.6, 10.2, 12.0, 13.3, 14.5, 15.0, 19.5, and 22.1.

In certain embodiments, the crystal form I of the compound represented by formula (I) has characteristic peaks at 5.2, 7.2, 7.9, 9.6, 10.2, 12.0, 13.3, 14.5, 15.0, 19.5, 21.2, 22.1, and 23.6. In certain embodiments, the X-ray powder diffraction pattern expressed in terms of 2θ angles, which are diffraction angles, of the crystal form I of the compound represented by formula (I) is shown in FIG. 10.

The present disclosure further provides a method for preparing the crystal form I of the compound represented by formula (I), the method comprising: a) mixing the compound represented by formula I with ethanol, dissolving, and heating, and b) crystallizing by cooling. In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form I of the compound represented by formula (I) of the present disclosure further comprises a filtration step, a washing step, a drying step, or the like.

The present disclosure provides a crystal form J of the compound represented by formula (I), and an X-ray powder diffraction pattern expressed in terms of 2θ angles, which are diffraction angles, of the crystal form has characteristic peaks at 7.7, 11.7, 13.5, 14.0, 18.6, 21.6, and 24.3. In certain embodiments, the crystal form J of the compound represented by formula (I) has characteristic peaks at 7.7, 10.1, 11.7, 13.5, 14.0, 18.6, 20.2, 21.6, 23.3, and 24.3.

In certain embodiments, the crystal form J of the compound represented by formula (I) has characteristic peaks at 7.7, 10.1, 10.7, 11.7, 13.5, 14.0, 16.0, 17.3, 18.6, 20.2, 21.6, 23.3, and 24.3.

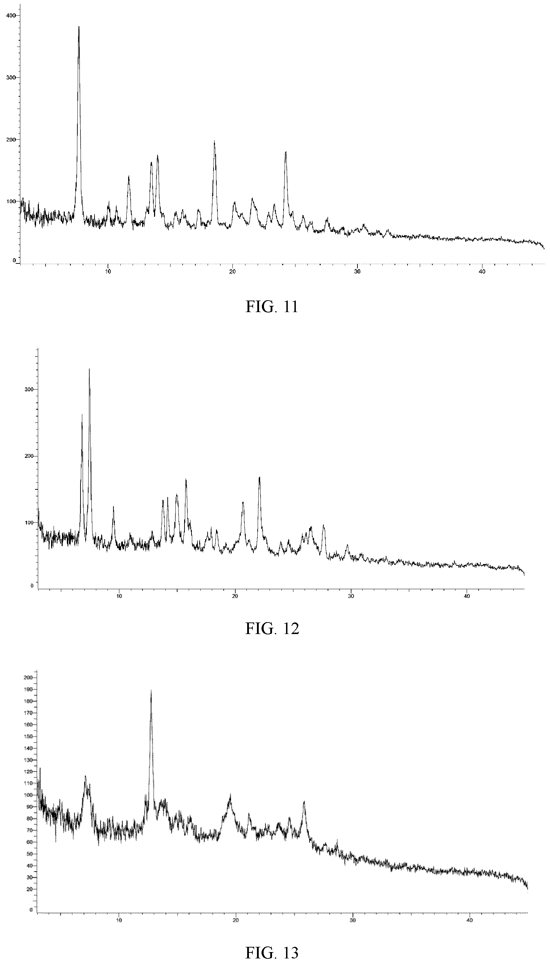

In certain embodiments, the X-ray powder diffraction pattern expressed in terms of 2θ angles, which are diffraction angles, of the crystal form J of the compound represented by formula (I) is shown in FIG. 11.

The present disclosure further provides a method for preparing the crystal form J of the compound represented by formula (I), the method comprising: a) mixing the crystal form A of the compound represented by formula I with isopropanol and heating, and b) crystallizing by cooling.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form J of the compound represented by formula (I) of the present disclosure further comprises a filtration step, a washing step, a drying step, or the like.

The present disclosure provides a crystal form K of the compound represented by formula (I), and an X-ray powder diffraction pattern expressed in terms of 2θ angles, which are diffraction angles, of the crystal form has characteristic peaks at 6.8, 7.4, 13.8, 15.0, 15.8, 20.7, and 22.1.

In certain embodiments, the crystal form K of the compound represented by formula (I) has characteristic peaks at 6.8, 7.4, 9.5, 13.8, 14.2, 15.0, 15.8, 20.7, 22.1, and 27.6.

In certain embodiments, the crystal form K of the compound represented by formula (I) has characteristic peaks at 6.8, 7.4, 9.5, 13.8, 14.2, 15.0, 15.8, 17.9, 18.4, 20.7, 22.1, 26.5, and 27.6. In certain embodiments, the X-ray powder diffraction pattern expressed in terms of 2θ angles, which are diffraction angles, of the crystal form K of the compound represented by formula (I) is shown in FIG. 12.

The present disclosure further provides a method for preparing the crystal form K of the compound represented by formula (I), the method comprising: a) mixing the crystal form A of the compound represented by formula I with acetonitrile and heating, and b) crystallizing by cooling.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form K of the compound represented by formula (I) of the present disclosure further comprises a filtration step, a washing step, a drying step, or the like.

The present disclosure provides a crystal form L of the compound represented by formula (I), and an X-ray powder diffraction pattern expressed in terms of 2θ angles, which are diffraction angles, of the crystal form has characteristic peaks at 7.1, 7.6, 12.8, 13.6, 19.5, 21.1, and 25.8.

In certain embodiments, the crystal form L of the compound represented by formula (I) has characteristic peaks at 7.1, 7.6, 9.2, 12.8, 13.6, 16.1, 19.5, 21.1, 24.6, and 25.8.

In certain embodiments, the crystal form L of the compound represented by formula (I) has characteristic peaks at 7.1, 7.6, 9.2, 12.8, 13.6, 16.1, 19.5, 21.1, 24.6, and 25.8.

In certain embodiments, the X-ray powder diffraction pattern expressed in terms of 2θ angles, which are diffraction angles, of the crystal form L of the compound represented by formula (I) is shown in FIG. 13.

The present disclosure further provides a method for preparing the crystal form L of the compound represented by formula (I), the method comprising: a) mixing the compound represented by formula I with dichloromethane and dissolving, and b) crystallizing by adding methyl tert-butyl ether.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form L of the compound represented by formula (I) of the present disclosure further comprises a filtration step, a washing step, a drying step, or the like.

The present disclosure provides a crystal form M of the compound represented by formula (I), and an X-ray powder diffraction pattern expressed in terms of 2θ angles, which are diffraction angles, of the crystal form has characteristic peaks at 6.6, 12.8, 14.8, 19.9, 25.3, 28.1, and 29.9. In certain embodiments, the crystal form M of the compound represented by formula (I) has characteristic peaks at 6.6, 12.8, 14.8, 15.3, 19.9, 24.9, 25.3, 25.9, 28.1, and 29.9.

In certain embodiments, the crystal form M of the compound represented by formula (I) has characteristic peaks at 6.6, 10.9, 12.8, 14.8, 15.3, 18.0, 19.9, 24.9, 25.3, 25.9, 27.0, 28.1, and 29.9.

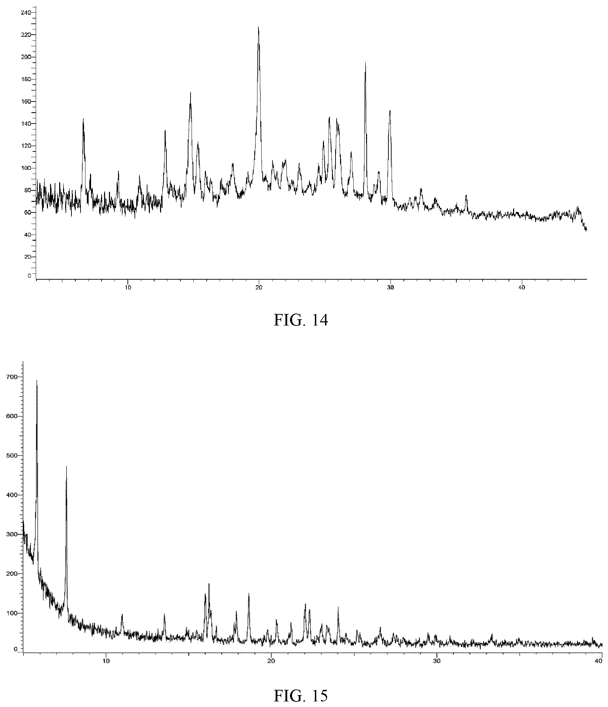

In certain embodiments, the X-ray powder diffraction pattern expressed in terms of 2θ angles, which are diffraction angles, of the crystal form M of the compound represented by formula (I) is shown in FIG. 14.

The present disclosure further provides a method for preparing the crystal form M of the compound represented by formula (I), the method comprising: a) mixing the compound represented by formula I with dimethyl sulfoxide and dissolving, and b) crystallizing by volatilization.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I.

In certain embodiments, the method for preparing the crystal form M of the compound represented by formula (I) of the present disclosure further comprises a filtration step, a washing step, a drying step, or the like.

The present disclosure provides a crystal form N of the compound represented by formula (I), and an X-ray powder diffraction pattern expressed in terms of 2θ angles, which are diffraction angles, of the crystal form has characteristic peaks at 5.8, 7.6, 16.0, 16.2, 18.6, 22.0, and 22.3.

In certain embodiments, the crystal form N of the compound represented by formula (I) has characteristic peaks at 5.8, 7.6, 13.5, 16.0, 16.2, 17.9, 18.6, 22.0, 22.3, and 24.0.

In certain embodiments, the crystal form N of the compound represented by formula (I) has characteristic peaks at 5.8, 7.6, 11.0, 13.5, 16.0, 16.2, 17.9, 18.6, 20.3, 21.2, 22.0, 22.3, and 24.0. In certain embodiments, the X-ray powder diffraction pattern expressed in terms of 2θ angles, which are diffraction angles, of the crystal form N of the compound represented by formula (I) is shown in FIG. 15.

The present disclosure further provides a method for preparing the crystal form N of the compound represented by formula (I), the method comprising: a) mixing the compound represented by formula I with a phosphoric acid solution and a solvent II, and heating to completely dissolve the compound, wherein the solvent II is selected from the group consisting of at least one of isopropanol, tetrahydrofuran, and ethanol, and b) crystallizing by cooling.

In certain embodiments, the volume (µL) of the solvent of the present disclosure may be 1 time to 200 times the mass (mg) of the compound of formula I; in non-limiting embodiments, the volume (µL) of the solvent of the present disclosure may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, or 200 times the mass (mg) of the compound of formula I. In certain embodiments, the method for preparing the crystal form N of the present disclosure further comprises a filtration step, a washing step, a drying step, or the like.

The present disclosure also provides a pharmaceutical composition prepared from an aforementioned crystal form of the compound represented by formula (I).

The present disclosure also provides a pharmaceutical composition comprising an aforementioned crystal form and optionally a pharmaceutically acceptable carrier, diluent, or excipient.

The present disclosure also provides a preparation method for a pharmaceutical composition, the preparation method comprising a step of mixing an aforementioned crystal form with a pharmaceutically acceptable carrier, diluent, or excipient.

The present disclosure also provides use of the aforementioned crystal forms, or the aforementioned composition, or a composition prepared by the aforementioned method in preparing a medicament for preventing and/or treating a PDE-related disorder.

The present disclosure also provides use of the aforementioned crystal forms, or the aforementioned composition, or a composition prepared by the aforementioned method in preparing a medicament for preventing and/or treating asthma, obstructive pulmonary disease, sepsis, nephritis, diabetes, allergic rhinitis, allergic conjunctivitis, ulcerative enteritis, or rheumatism.

As used herein, "2θ or 2θ angle" refers to a diffraction angle, and θ is the Bragg angle in ° or degrees; the 2θ of each characteristic peak has a margin of error of ±0.20 (including the case that a number having more than 1 decimal place is rounded), and the margin of error may be -0.20, - 0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

According to "9103 Guidelines for Hygroscopicity of Medicaments" in Volume IV of the 2015 edition of the Chinese Pharmacopoeia, hygroscopicity characteristics and hygroscopic weight gains are defined as follows:
deliquescent: sufficient water is absorbed to form a liquid;
extremely hygroscopic: the hygroscopic weight gain is not less than 15%;
hygroscopic: the hygroscopic weight gain is less than 15% but not less than 2%;
slightly hygroscopic: the hygroscopic weight gain is less than 2% but not less than 0.2%;
non-hygroscopic or almost non-hygroscopic: the hygroscopic weight gain is less than 0.2%.

As used herein, "differential scanning calorimetry" or "DSC" refers to the measurement of the temperature difference and heat flow difference between a sample and a reference substance during a ramping or thermostatic process to characterize all the physical changes and chemical changes related to the thermal effect, and to obtain the phase change information about the sample.

In the present disclosure, the drying temperature is generally 25 °C to 150 °C, preferably 40 °C to 80 °C, and the drying may be performed under atmospheric pressure or reduced pressure.

"Pharmaceutical composition" refers to a mixture containing one or more of the compound of formula (I) or the pharmaceutically acceptable salt thereof described herein, and other chemical components, as well as other components, such as pharmaceutically acceptable carriers and excipients. The pharmaceutical composition is intended to promote administration to an organism and facilitate the absorption of the active ingredient so that it can exert its biological activity.

The crystal forms of the present disclosure include, but are not limited to, a solvate of the compound represented by formula (I), and the solvents include, but are not limited to, n-heptane, cyclohexane, petroleum ether, dichloromethane, acetonitrile, methanol, propylene glycol methyl ether, 1,2-dichloroethane, ethyl acetate, ethanol, n-propanol, tetrahydrofuran, trichloromethane, N,N-dimethylformamide, acetone, isopropyl acetate, methyl *tert*-butyl ether, *N-*methylpyrrolidone, water, 2-butanone, 1,4-dioxane, isoamyl alcohol, o-xylene, isopropyl ether, toluene, isopropanol, dimethyl sulfoxide, and benzyl alcohol.

As used herein, "solvate" includes, but is not limited to, a complex formed by combining the compound of formula (I) with a solvent.

### BRIEF DESCRIPTION OF THE DRAWINGS

FIG. 1: an XRPD pattern of the amorphous form of the compound represented by formula (I).
FIG. 2: an XRPD pattern of the crystal form A of the compound represented by formula (I).
FIG. 3: an XRPD pattern of the crystal form B of the compound represented by formula (I).
FIG. 4: an XRPD pattern of the crystal form C of the compound represented by formula (I).
FIG. 5: an XRPD pattern of the crystal form D of the compound represented by formula (I).
FIG. 6: an XRPD pattern of the crystal form E of the compound represented by formula (I).
FIG. 7: an XRPD pattern of the crystal form F of the compound represented by formula (I).
FIG. 8: an XRPD pattern of the crystal form G of the compound represented by formula (I).
FIG. 9: an XRPD pattern of the crystal form H of the compound represented by formula (I).
FIG. 10: an XRPD pattern of the crystal form I of the compound represented by formula (I).
FIG. 11: an XRPD pattern of the crystal form J of the compound represented by formula (I).
FIG. 12: an XRPD pattern of the crystal form K of the compound represented by formula (I).
FIG. 13: an XRPD pattern of the crystal form L of the compound represented by formula (I).
FIG. 14: an XRPD pattern of the crystal form M of the compound represented by formula (I).
FIG. 15: an XRPD pattern of the crystal form N of the compound represented by formula (I).

### DETAILED DESCRIPTION

The present disclosure is explained below in more detail with reference to examples or test examples. The examples or test examples of the present disclosure are only illustrative of the technical solutions of the present disclosure and do not limit the essence and scope of the present disclosure.

Experimental methods without conditions specified in the examples of the present disclosure were generally conducted under conventional conditions or conditions recommended by the manufacturers of the starting materials or commercial products. Reagents without specific origins indicated were commercially available conventional reagents.

The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument, with dimethyl sulfoxide-D6 (DMSO-*d₆*), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters; MS model: Waters ACQuity Qda Detector/Waters SQ Detector), and THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and a Waters HPLC e2695-2489 high-pressure liquid chromatograph.

The chiral HPLC analyses were performed using an Agilent 1260 DAD high performance liquid chromatograph.

The preparative high performance liquid chromatography steps were performed using Waters 2767, Waters 2767-SQ Detecor2, Shimadzu LC-20AP, and a Gilson-281 preparative chromatograph.

The preparative chiral chromatography steps were performed using a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm to 0.2 mm, and those used in the thin-layer chromatography separation and purification had a layer thickness of 0.4 mm to 0.5 mm.

In the silica gel column chromatography, a 200-300 mesh silica gel (Huanghai, Yantai) was generally used as the carrier.

The kinase mean inhibition rates and IC₅₀ values were measured using a NovoStar microplate reader (BMG, Germany).

The known starting materials in the present disclosure may be synthesized by using or according to methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling. The microwave reactions were performed using a CEM Discover-S 908860 microwave reactor. In the examples, the solutions were aqueous solutions unless otherwise specified.

In the examples, the reaction temperature was room temperature, i.e., 20 °C to 30 °C, unless otherwise specified.

The reaction progress monitoring in the examples was performed using thin-layer chromatography (TLC). The developing solvents used in the reactions, the eluent systems used in the column chromatography purification of the compounds, and the developing solvent systems used in the thin-layer chromatography include: A: a n-hexane/ethyl acetate system, and B: a dichloromethane/methanol system. The volume ratio of the solvents was adjusted depending on the polarity of the compounds, or by adding a small amount of basic or acidic reagents such as triethylamine and acetic acid.

Test conditions for the instruments used in the experiments in the present disclosure:
1. Differential scanning calorimeter (DSC)
   Instrument model: Mettler Toledo DSC 3+STARe System
   Gas purging: nitrogen gas; nitrogen purging speed: 50 mL/min
   Ramp rate: 10.0 °C/min
   Temperature range: 25 °C to 350 °C (or 25 °C to 300°C)
2. X-ray powder diffraction (XRPD)
   Instrument model: BRUKER D8 Discover X-ray powder diffractometer
   Ray: monochromatic Cu-Kα ray (λ = 1.5418 Å)
   Scan mode: θ/2θ, scan range (2θ range): 3° to 45°
   Voltage: 40 kV, current: 40 mA
3. Thermogravimetric analysis (TGA)
   Instrument model: Mettler Toledo TGA2
   Gas purging: nitrogen gas; nitrogen purging speed: 50 mL/min
   Ramp rate: 10.0 °C/min
   Temperature range: 30 °C to 350 °C
   **Example 1:** Preparation of Compound Represented by Formula (I)

### Preparation of 9,10-dimethoxy-2-[[2-(2-oxo-imidazolidin-1-yl)-ethyl]-(2,4,6-trimethyl-phenyl)-amino]-6,7-dihydro-pyrimido[6,1-a]-isoquinolin-4-one (Compound 1)

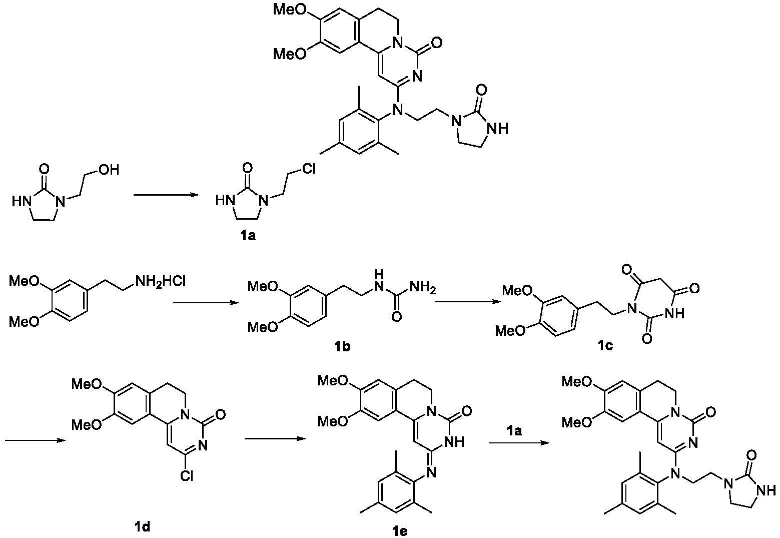

### Preparation of intermediate 1a: 1-(2-chloroethyl)imidazolidinone

Thionyl chloride (5 mL) was slowly added to 1-(2-hydroxyethyl)imidazolidinone (3.5 g, 26.9 mmol) at 0 °C. The mixture was heated to 45 °C and stirred until the reaction was completed. A saturated sodium chloride solution was added to quench the reaction, and the pH was adjusted to 7 with a 10% NaOH solution. The resulting mixture was extracted with dichloromethane, washed with a saturated sodium chloride solution, and dried over anhydrous sodium sulfate. The filtrate was concentrated under reduced pressure to give intermediate 1a (3.5 g, 88.4% yield), MS(ESI) m/z 149.1 [M+H]⁺.

### Preparation of intermediate 1b: 1-(3,4-dimethoxyphenethyl)urea

2-(3,4-Dimethoxyphenyl)ethylamine hydrochloride (4.3 g, 19.8 mmol) was dissolved in water (25 mL) at room temperature, and the solution was heated to 50 °C. Potassium cyanate (1.8 g, 21.8 mmol) was added in batches, and the resulting mixture was stirred until the reaction was completed. The reaction mixture was cooled to 0 °C and filtered. The filter cake was washed with ice-cold water and dried to give intermediate 1b (4.1 g, 93.8% yield), MS(ESI) m/z 225.1 [M+H]⁺.

### Preparation of intermediate 1c: 1-[2-(3,4-dimethoxy-phenyl)-ethyl]-pyrimidine-2,4,6-trione

Sodium ethoxide (3.8 g, 55.8 mmol) was added in batches to absolute ethanol (50 mL) in an ice bath. After the addition, the mixture was heated at reflux, and diethyl malonate (5.9 g, 36.6 mmol) was added dropwise. After the addition, the resulting mixture was stirred for 0.25 h to 0.5 h. A solution of intermediate 1b (4.1 g, 18.3 mmol) in ethanol (30 mL) was added dropwise, and the mixture was stirred until the reaction was completed. The reaction mixture was cooled to 0 °C, and a 5% HCl solution was added dropwise until the pH was 6. Water (300 mL) was added, and the mixture was filtered. The filter cake was washed with ice-cold water and dried to give intermediate 1c (3.9 g, 77.1% yield), MS(ESI) m/z 293.1 [M+H]⁺.

### Preparation of intermediate 1d: 2-chloro-9,10-dimethoxy-6,7-dihydropyrimido[6,1-a]isoquinolin-4-one

Intermediate B2 (3.9 g, 13.4 mmol) was added to phosphorus oxychloride (120 mL) at room temperature. The mixture was heated to 110 °C and stirred until the reaction was completed. The reaction mixture was cooled and concentrated, and the solid was poured into ice-cold water. A saturated NaOH solution was added dropwise until the pH was 10, and the resulting mixture was filtered. The filter cake was washed with ice-cold water and dried to give intermediate 1d (2.4 g, 62.4% yield), MS(ESI) m/z 293.1 [M+H]⁺.

### Preparation of intermediate 1e: 9,10-dimethoxy-2-(2,4,6-trimethyl-phenylimino)-2,3,6,7-tetrahydropyrimido[6,1-a]isoquinolin-4-one

Intermediate 1d (2.4 g, 8.2 mmol) was suspended in isopropanol (30 mL) at room temperature, and 2,4,6-trimethylaniline (4.5 g, 24.6 mmol) was added. The system was heated to 90 °C and stirred until the reaction was completed. The reaction mixture was cooled and filtered. The filter cake was washed with ice-cold water and dried to give intermediate 1e (3.0 g, 92.1% yield), MS(ESI) m/z 392.2 [M+H]⁺.

### Preparation of compound 1: 9,10-dimethoxy-2-[[2-(2-oxo-imidazolidin-1-yl)-ethyl]-(2,4,6-trimethyl-phenyl)-amino]-6,7-dihydro-pyrimido[6,1-a]isoquinolin-4-one

Intermediate 1e (0.72 g, 1.8 mmol) was dissolved in tetrahydrofuran (20 mL) at room temperature, and potassium tert-butoxide (0.42 g, 3.6 mmol) was added under a nitrogen atmosphere. After the addition, the mixture was heated to 65 °C, stirred for 48 h, and cooled to 25 °C, and intermediate 1a (0.82 g, 5.5 mmol) was added. After the addition, the resulting mixture was heated to 80 °C and stirred until the reaction was completed. A saturated sodium chloride solution was added to quench the reaction, and the reaction mixture was extracted with dichloromethane, washed with a saturated sodium chloride solution, and dried over anhydrous sodium sulfate. The filtrate was concentrated under reduced pressure and purified by silica gel column chromatography (n-heptane/ethyl acetate) to give the target compound 1 (0.21 g, 46.5% yield).

¹H NMR (400 MHz, CDCl₃) *δ* 6.99 (s, 2H), 6.69 (s, 1H), 6.64 (s, 1H), 5.39 (s, 1H), 4.61 (s, 1H), 4.22-4.15 (m, 2H), 4.08-3.99 (m, 2H), 3.93 (s, 3H), 3.77-3.69 (m, 5H), 3.55-3.46 (m, 2H), 3.38-3.42 (t, J = 6.8 Hz, 2H), 2.88-2.92 (t, J = 6.4 Hz, 2H), 2.34 (s, 3H), 2.18 (s, 6H).

MS(ESI) m/z 504.4 [M+H]⁺.

### Comparative Example 1

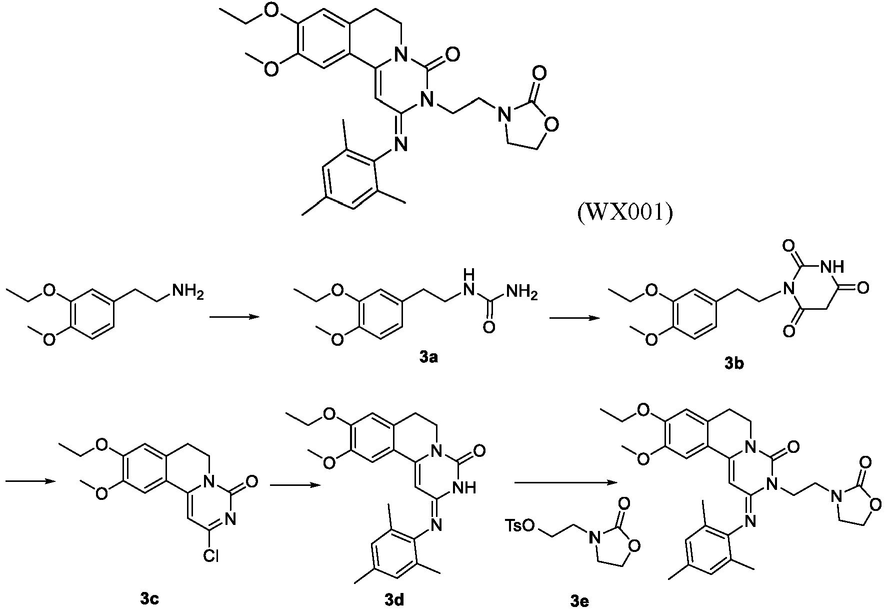

Intermediate 3d was prepared according to the method of Example 1 using 2-(3-ethoxy-4-methoxyphenyl)ethylamine hydrochloride as a starting material.

Intermediate 3d (1 g) was dissolved in 1,2-dichloroethane (20 mL) at room temperature, and 2-(2-oxooxazolidin-3-yl)ethyl 4-methylbenzenesulfonate (844 mg), potassium carbonate (612 mg), and sodium iodide (443 mg) were sequentially added. The mixture was heated to 80 °C and stirred until the reaction was completed. The reaction mixture was cooled, filtered, concentrated, diluted with water, and extracted with ethyl acetate. The organic phases were combined, dried, filtered, concentrated, and purified by column chromatography to give WX001. MS(ESI) m/z 519.0 [M+H]⁺.

### Biological Evaluations

The present disclosure is further described and explained below using test examples. However, these test examples are not intended to limit the scope of the present disclosure.

### Test Example 1: In Vitro PDE4B Enzyme Activity Assay: An IMAP FP-Based Analysis Method 1. Materials

| Material | Brand | Cat. No./model |
|---|---|---|
| PDE4B1 | BPS | 60041 |
| Trequinsin | Sigma | T2057 |
| 384-well plate | Perkin Elmer | 6007279 |
| IMAP FP IPP assay kit | MOLECULAR DEVICES | R8124 |

### 2. Procedures

The compound was serially diluted 5-fold with DMSO to different concentrations (10,000 nM, 2000 nM, 400 nM, 80 nM, 16 nM, 3.2 nM, 0.64 nM, 0.128 nM, 0.0256 nM, and 0.005 nM). 200 µL of the compound at different concentrations was added to a 384-well plate (n = 2), and meanwhile, two 200-µL parts of DMSO were added to the 384-well plate (n = 2) as blank controls. Then 10 µL of a 0.025 µg/mL PDE4B1 enzyme solution (prepared with 1 mM 5*IMAP reaction buffer and 1 mM DTT) was added to the 384-well plate, and 10 µL of PDE4B1 enzyme-free blank buffer was added to one of the blank controls. The plate was incubated with shaking at room temperature for 15 min, and then 10 µL of a 0.1 µM FAM-cAMP solution (prepared with 1 mM 5*IMAP reaction buffer and 1 mM DTT) was added to the plate. After the plate was incubated with shaking at room temperature for 30 min, 60 µL of an assay solution (prepared with 0.5625 mM 5*IMAP progressive binding buffer A, 0.1875 mM 5*IMAP progressive binding buffer B, and 0.75 mM beads) was added. After the plate was incubated with shaking at room temperature for 60 min, data were collected. Inhibition rates were calculated by the formula: inhibition rate = M/(M - M_{control}) × 100; the IC₅₀ value was calculated from a fitted curve of concentrations vs. inhibition rates. RPL554 was used as a positive control in this assay. The *in vitro* inhibition of PDE4B1 enzyme activity by the example of the present disclosure was assessed through the above assay, and the calculated IC₅₀ values are shown in Table I and Table II.

### Test Example 2: In Vitro PDE3A Enzyme Activity Assay: An IMAP FP-Based Analysis Method

### 1. Materials

| Material | Brand | Cat. No./model |
|---|---|---|
| PDE3A | BPS | 60030 |
| Trequinsin | Sigma | T2057 |
| 384-well plate | Perkin Elmer | 6007279 |
| IMAP FP IPP assay kit | MOLECULAR DEVICES | R8124 |

### 2. Procedures

The compound was serially diluted 5-fold with DMSO to different concentrations (10,000 nM, 2000 nM, 400 nM, 80 nM, 16 nM, 3.2 nM, 0.64 nM, 0.128 nM, 0.0256 nM, and 0.005 nM). 200 µL of the compound at different concentrations was added to a 384-well plate (n = 2), and meanwhile, two 200-µL parts of DMSO were added to the 384-well plate (n = 2) as blank controls. Then 10 µL of a 0.025 µg/mL PDE4B1 enzyme solution (prepared with 1 mM 5*IMAP reaction buffer and 1 mM DTT) was added to the 384-well plate, and 10 µL of PDE3A enzyme-free blank buffer was added to one of the blank controls. The plate was incubated with shaking at room temperature for 15 min, and then 10 µL of a 0.1 µM FAM-cAMP solution (prepared with 1 mM 5*IMAP reaction buffer and 1 mM DTT) was added to the plate. After the plate was incubated with shaking at room temperature for 30 min, 60 µL of an assay solution (prepared with 0.5625 mM 5*IMAP progressive binding buffer, 0.1875 mM 5*IMAP progressive binding buffer B, and 0.75 mM beads) was added. After the plate was incubated with shaking at room temperature for 60 min, data were collected. Inhibition rates were calculated by the formula: inhibition rate = M/(M - M_{control}) × 100; the IC₅₀ value was calculated from a fitted curve of concentrations vs. inhibition rates. RPL554 was used as a positive control in this assay.

The *in vitro* inhibition of PDE3A enzyme activity by the example of the present disclosure was assessed through the above assay, and the calculated IC₅₀ values are shown in Table I and Table II.

**Table I**

| No. | Inhibition rate (%) | |
|---|---|---|
| | PDE3A (500 nM) | PDE4B1(2000 nM) |
| RPL554 | 99.2 | 64.2 |
| Compound 1 | 100.3 | 82.8 |
| WX001 | 98.6 | 66.3 |

**Table II**

| No. | IC₅₀ (nM) | |
|---|---|---|
| | PDE3A | PDE4B1 |
| RPL554 | 0.25 | 110 |
| Compound 1 | 0.13 | 50 |
| WX001 | 0.90 | 876 |

| | | |
|---|---|---|
| Note: N/A stands for undetected. | | |

Conclusion: Compared with the positive compound RPL554, compound 1 showed good biological activity in the *in vitro* enzyme assay, and compound 1 has 7 times higher inhibitory activity against PDE3A enzyme than compound WX001, showing good development prospects.

### Test Example 3: PK Assay by Intratracheal Administration

### 1. Objective

To evaluate the pharmacokinetic profiles of test samples in SD rats and their distributions in lung tissue after intratracheal administration.

### 2. Test protocol

### 2.1. Test compounds

### Compound 1 and RPL-554

### 2.2. Test animals

198 ICR mice (Shanghai Slac Laboratory Animal Co., Ltd.), half being male and half female.

### 2.3. Compound solution preparation

### 1) Homogeneous solution:

0.5 g of tween 80 was weighed out and dissolved in 50 mL of a pH 2.5 citric acid/disodium hydrogen phosphate buffered salt solution to obtain a solution for later use.

1.0 mg of test compound was weighed out and dissolved in an appropriate amount of the tween solution to prepare a 0.03 mg/mL solution for later use.

### 2) Suspension:

0.5 g of CMC-Na and 0.5 g of tween 20 were weighed out and well stirred with 50 mL of a 0.9% normal saline solution to obtain a 1% CMC-Na and tween 20 solution for later use.

1.0 mg of test compound was weighed out, added to 10 mL of the aforementioned solution, and dispersed ultrasonically, and the mixture was well stirred to obtain a suspension for later use.

### 2.4. Administration regimens

| | Regimen 1 (suspension) | Regimen 2 (homogeneous solution) |
|---|---|---|
| Concentration | 0.15 mg/mL | 0.03 mg/mL |
| Volume | 40µL | 40µL |
| Dose | 6 µg/mouse | 1.2 µg/mouse |

### 3. Procedures/process

### 3.1. Intratracheal administration to mice

Mice were anesthetized with isoflurane gas and then administered compounds via the trachea. Time points for plasma collection were: 0.25 h, 0.5 h, 1 h, 2 h, 4 h, 8 h, 12 h, and 24 h. 200 µL of whole blood was collected, then anticoagulated with EDTA-K2, and centrifuged at 2 °C to 8 °C at a rate of about 6800 g for 6 min. The resulting plasma was transferred to properly labeled test tubes within 1 h of blood collection/centrifugation and stored at -80 °C. Time points for lung tissue collection were: 0.5 h, 2 h, 8 h, and 24 h. The collected tissue samples were transferred to properly labeled test tubes and stored at -80 °C.

### 3.2. Plasma treatment and LC-MS/MS analysis

30.0 µL of plasma sample was added to a 1.5 mL centrifuge tube, and 150 µL of an internal standard working solution was added. The mixture was vortexed for 1 min and centrifuged for 5 min (13,000 rpm, 4 °C). 70.0 µL of the supernatant was added to a 96-well plate, and 70.0 µL of deionized water was added. The resulting mixture was well shaken, and 2.00 µL of the mixture was taken for LC-MS/MS analysis.

### 3.3. Lung tissue treatment

An appropriate amount of the lung tissue sample was accurately weighed out and placed into a homogenate tube, and acetonitrile was added at a volume equivalent to 5 times the weight of the sample. The mixture was homogenized and ultrasonicated for 5 min. 20.0 µL of the lung tissue homogenate sample was taken, and 30.0 µL of an internal standard working solution and 200 µL of acetonitrile were added. The mixture was vortexed for 1 min and centrifuged for 10 min (4000 rpm, 4 °C). 100 µL of the supernatant was added to a 96-well plate, and 100 µL of deionized water was added. The resulting mixture was well mixed by shaking the plate (1000 rpm, RT), and 1.00 µL of the mixture was taken for LC-MS/MS analysis.

### 4. Pharmacokinetic parameters

Compared with RPL-554, compound 1 showed relatively high exposure *in vivo* and relatively long retention in the lung. The related data are shown in Tables III and IV.

**Table III: The PK and tissue distribution of the suspension formula**

| | Plasma | | | | Lung | | | |
|---|---|---|---|---|---|---|---|---|
| | Tₘₐₓ (h) | Cₘₐₓ (ng/mL) | AUC_{0-∞} (h*ng/mL) | T_{1/2} (h) | Tₘₐₓ (h) | Cₘₐₓ (ng/mL) | AUC_{0-∞} (h*ng/mL) | T_{1/2} (h) |
| RPL-554 | 0.25 | 9.55 | 8.19 | 0.88 | 24 | 5090 | 75300 | / |
| Compound 1 | 0.25 | 21.9 | 27.9 | 39.9 | 0.5 | 10700 | 126000 | 12.6 |

**Table IV: The PK and tissue distribution of the homogeneous solution formula**

| | Plasma | | | | Lung | | |
|---|---|---|---|---|---|---|---|
| | Tₘₐₓ (h) | Cₘₐₓ (ng/mL) | AUC_{0-∞} (h*ng/mL) | T_{1/2} (h) | Tₘₐₓ (h) | Cₘₐₓ (ng/mL) | AUC_{0-∞} (h*ng/mL) |
| RPL-554 | 0.25 | 5.77 | 2.1 | / | 0.5 | 35.1 | 38.5 |
| Compound 1 | 0.25 | 11.9 | 5.54 | 0.377 | 0.5 | 220 | 2040 |

### Example 2: Preparation of Amorphous Form of Compound Represented by Formula (I)

About 8 mg of the compound of formula (I) was taken, and 0.04 mL of benzyl alcohol was added. The compound was dissolved by stirring at room temperature, and crystallization was performed by volatilization at room temperature to give a product. According to X-ray powder diffraction analysis, the product was the amorphous form.

### Example 3: Preparation of Crystal Form A of Compound Represented by Formula (I)

A product was obtained according to the preparation in Example 1. According to X-ray powder diffraction analysis, the product was defined as crystal form A. Its XRPD pattern is shown in FIG. 2, and its characteristic peak positions are shown in Table 1. Its DSC thermogram shows endothermic peaks at 135.93 °C and 263.19 °C. Its TGA thermogram shows a weight loss of 3.97% between 30 °C and 275 °C.

**Table 1**

| **Peak No.** | **2θ value [° or degrees]** | **d[Å]** | **Relative intensity%** |
|---|---|---|---|
| 1 | 4.560 | 19.36247 | 35.8 |
| 2 | 6.331 | 13.95022 | 53.0 |
| 3 | 6.640 | 13.30158 | 62.3 |
| 4 | 8.265 | 10.68985 | 11.6 |
| 5 | 9.251 | 9.55157 | 23.6 |
| 6 | 10.071 | 8.77605 | 34.2 |
| 7 | 10.963 | 8.06422 | 100.0 |
| 8 | 11.374 | 7.77368 | 19.3 |
| 9 | 11.822 | 7.48011 | 24.9 |
| 10 | 12.770 | 6.92656 | 26.0 |
| 11 | 13.084 | 6.76117 | 32.9 |
| 12 | 13.534 | 6.53717 | 9.1 |
| 13 | 14.358 | 6.16388 | 45.4 |
| 14 | 15.530 | 5.70140 | 50.5 |
| 15 | 16.024 | 5.52673 | 31.9 |
| 16 | 16.589 | 5.33977 | 22.7 |
| 17 | 17.076 | 5.18833 | 25.7 |
| 18 | 17.407 | 5.09061 | 19.8 |
| 19 | 17.927 | 4.94412 | 78.9 |
| 20 | 18.810 | 4.71387 | 30.1 |
| 21 | 19.122 | 4.63755 | 71.8 |
| 22 | 20.201 | 4.39228 | 10.6 |
| 23 | 22.817 | 3.89432 | 34.4 |
| 24 | 23.734 | 3.74581 | 40.0 |
| 25 | 24.211 | 3.67319 | 17.4 |
| 26 | 24.648 | 3.60899 | 20.5 |
| 27 | 25.871 | 3.44104 | 26.6 |
| 28 | 26.833 | 3.31981 | 17.8 |
| 29 | 28.633 | 3.11511 | 9.0 |
| 30 | 29.411 | 3.03449 | 6.3 |
| 31 | 30.966 | 2.88555 | 6.6 |
| 32 | 32.282 | 2.77085 | 3.4 |

### Example 4: Preparation of Crystal Form B of Compound Represented by Formula (I)

About 8 mg of the compound of formula (I) was taken, and 0.16 mL of dichloromethane was added. The compound was dissolved by stirring at room temperature, and crystallization was performed by volatilization at room temperature to give a product. According to X-ray powder diffraction analysis, the product was defined as crystal form B. Its XRPD pattern is shown in FIG. 3, and its characteristic peak positions are shown in Table 2. Its DSC thermogram shows endothermic peaks at 139.31 °C and 263.02 °C and an exothermic peak at 153.33 °C. Its TGA thermogram shows a weight loss of 7.14% between 30 °C and 290 °C.

**Table 2**

| **Peak No.** | **2θ value [° or degrees]** | **d[Å]** | **Relative intensitv%** |
|---|---|---|---|
| 1 | 6.579 | 13.42447 | 79.6 |
| 2 | 7.020 | 12.58171 | 68.6 |
| 3 | 8.563 | 10.31738 | 22.9 |
| 4 | 10.001 | 8.83689 | 37.9 |
| 5 | 10.950 | 8.07349 | 100.0 |
| 6 | 11.734 | 7.53560 | 28.5 |
| 7 | 12.701 | 6.96423 | 58.0 |
| 8 | 15.482 | 5.71890 | 90.1 |
| 9 | 16.084 | 5.50628 | 31.3 |
| 10 | 16.503 | 5.36729 | 22.1 |
| 11 | 17.487 | 5.06752 | 22.6 |
| 12 | 17.823 | 4.97254 | 23.5 |
| 13 | 19.142 | 4.63281 | 42.8 |
| 14 | 19.381 | 4.57632 | 20.7 |
| 15 | 20.236 | 4.38469 | 11.6 |
| 16 | 21.134 | 4.20038 | 19.6 |
| 17 | 22.397 | 3.96634 | 15.3 |
| 18 | 23.379 | 3.80190 | 10.9 |
| 19 | 23.709 | 3.74978 | 40.2 |
| 20 | 24.221 | 3.67164 | 34.5 |
| 21 | 24.614 | 3.61392 | 39.6 |
| 22 | 25.820 | 3.44771 | 45.6 |
| 23 | 26.129 | 3.40768 | 21.0 |
| 24 | 26.859 | 3.31675 | 20.9 |
| 25 | 28.570 | 3.12181 | 14.1 |
| 26 | 31.218 | 2.86279 | 5.2 |

### Example 5: Preparation of Crystal Form C of Compound Represented by Formula (I)

About 8 mg of the compound of formula (I) was taken, and 0.28 mL of acetonitrile/methanol (1:1) was added. The compound was dissolved by stirring at room temperature, and crystallization was performed by volatilization at room temperature to give a product. According to X-ray powder diffraction analysis, the product was defined as crystal form C. Its XRPD pattern is shown in FIG. 4, and its characteristic peak positions are shown in Table 3. Its DSC thermogram shows endothermic peaks at 148.77 °C and 262.90 °C. Its TGA thermogram shows a weight loss of 4.36% between 30 °C and 195 °C.

**Table 3**

| **Peak No.** | **2θ value [° or degrees]** | **d[Å]** | **Relative intensity%** |
|---|---|---|---|
| 1 | 6.633 | 13.31444 | 19.5 |
| 2 | 7.478 | 11.81264 | 19.4 |
| 3 | 10.067 | 8.77917 | 16.8 |
| 4 | 10.909 | 8.10399 | 100.0 |
| 5 | 13.819 | 6.40302 | 8.2 |
| 6 | 15.026 | 5.89153 | 5.4 |
| 7 | 15.550 | 5.69395 | 11.0 |
| 8 | 16.557 | 5.34979 | 3.9 |
| 9 | 17.486 | 5.06763 | 6.9 |
| 10 | 17.887 | 4.95506 | 6.4 |
| 11 | 18.444 | 4.80659 | 5.0 |
| 12 | 19.301 | 4.59512 | 6.3 |
| 13 | 20.321 | 4.36671 | 4.5 |
| 14 | 20.693 | 4.28891 | 7.6 |
| 15 | 22.099 | 4.01924 | 4.6 |
| 16 | 23.811 | 3.73393 | 10.9 |
| 17 | 24.315 | 3.65767 | 11.8 |
| 18 | 24.705 | 3.60077 | 8.7 |
| 19 | 25.817 | 3.44819 | 3.1 |
| 20 | 26.169 | 3.40260 | 2.5 |
| 21 | 26.968 | 3.30358 | 7.3 |
| 22 | 27.631 | 3.22579 | 2.7 |

### Example 6: Preparation of Crystal Form D of Compound Represented by Formula (I)

About 8 mg of the compound of formula (I) was taken, and 0.8 mL of propylene glycol methyl ether was added. The mixture was heated to completely dissolve the compound, and crystallization was performed by volatilization at room temperature to give a product. According to X-ray powder diffraction analysis, the product was defined as crystal form D. Its XRPD pattern is shown in FIG. 5, and its characteristic peak positions are shown in Table 4. Its DSC thermogram shows endothermic peaks at 163.12 °C and 263.24 °C and an exothermic peak at 166.84 °C. Its TGA thermogram shows a weight loss of 3.02% between 30 °C and 165 °C.

**Table 4**

| **Peak No.** | **2θ value [° or degrees]** | **d[Å]** | **Relative intensitv%** |
|---|---|---|---|
| 1 | 6.633 | 13.31532 | 46.8 |
| 2 | 10.074 | 8.77347 | 42.3 |
| 3 | 10.965 | 8.06270 | 100.0 |
| 4 | 15.555 | 5.69210 | 19.8 |
| 5 | 16.117 | 5.49498 | 4.4 |
| 6 | 16.527 | 5.35938 | 10.8 |
| 7 | 17.488 | 5.06716 | 15.5 |
| 8 | 17.830 | 4.97054 | 12.4 |
| 9 | 19.320 | 4.59059 | 3.5 |
| 10 | 19.802 | 4.47995 | 2.3 |
| 11 | 20.274 | 4.37668 | 8.0 |
| 12 | 23.347 | 3.80709 | 5.5 |
| 13 | 23.747 | 3.74387 | 7.8 |
| 14 | 24.236 | 3.66944 | 10.2 |
| 15 | 24.700 | 3.60146 | 5.6 |
| 16 | 25.128 | 3.54115 | 5.5 |
| 17 | 25.847 | 3.44427 | 6.4 |
| 18 | 26.168 | 3.40268 | 4.7 |
| 19 | 26.934 | 3.30758 | 8.3 |
| 20 | 28.717 | 3.10624 | 3.7 |
| 21 | 29.467 | 3.02877 | 3.6 |
| 22 | 31.026 | 2.88005 | 2.0 |
| 23 | 31.395 | 2.84708 | 1.6 |
| 24 | 33.266 | 2.69111 | 1.7 |
| 25 | 34.116 | 2.62596 | 1.4 |

### Example 7: Preparation of Crystal Form D of Compound Represented by Formula (I)

About 8 mg of the compound of formula (I) was taken, and 0.44 mL of ethyl acetate/ethanol (1:1) was added. The compound was dissolved by stirring at room temperature, and crystallization was performed by volatilization at room temperature to give a product. According to X-ray powder diffraction analysis, the product was the crystal form D.

### Example 8: Preparation of Crystal Form D of Compound Represented by Formula (I)

About 8 mg of the compound of formula (I) was taken, and 0.3 mL of ethanol was added. The compound was dissolved by stirring at room temperature. 1 mL of water was added, and a solid precipitated. The mixture was centrifuged, and the solid was dried *in vacuo* to give a product. According to X-ray powder diffraction analysis, the product was the crystal form D.

### Example 9: Preparation of Crystal Form D of Compound Represented by Formula (I)

About 8 mg of the crystal form A of the compound of formula (I) was taken, and 0.8 mL of water was added. Crystallization was performed by stirring at room temperature. The mixture was centrifuged, and the solid was dried *in vacuo* to give a product. According to X-ray powder diffraction analysis, the product was the crystal form D.

### Example 10: Preparation of Crystal Form D of Compound Represented by Formula (I)

About 8 mg of the crystal form A of the compound of formula (I) was taken, and 0.8 mL of acetone was added. Crystallization was performed by stirring at room temperature. The mixture was centrifuged, and the solid was dried *in vacuo* to give a product. According to X-ray powder diffraction analysis, the product was the crystal form D.

### Example 11: Preparation of Crystal Form E of Compound Represented by Formula (I)

About 8 mg of the compound of formula (I) was taken, and 0.3 mL of N-methylpyrrolidone was added. The compound was dissolved by stirring. 1 mL of isopropyl acetate was added, and a solid precipitated. The mixture was centrifuged, and the solid was dried *in vacuo* to give a product. According to X-ray powder diffraction analysis, the product was defined as crystal form E. Its XRPD pattern is shown in FIG. 6, and its characteristic peak positions are shown in Table 5. Its DSC thermogram shows an endothermic peak at 265.03 °C. Its TGA thermogram shows a weight loss of 0.33% between 30 °C and 170 °C.

**Table 5**

| **Peak No.** | **2θ value [° or degrees]** | **d[Å]** | **Relative intensitv%** |
|---|---|---|---|
| 1 | 6.335 | 13.94117 | 100.0 |
| 2 | 11.087 | 7.97398 | 29.8 |
| 3 | 12.780 | 6.92145 | 33.3 |
| 4 | 13.019 | 6.79473 | 24.1 |
| 5 | 14.300 | 6.18857 | 53.8 |
| 6 | 15.890 | 5.57273 | 11.8 |
| 7 | 17.044 | 5.19814 | 18.7 |
| 8 | 17.915 | 4.94726 | 91.0 |
| 9 | 18.655 | 4.75264 | 12.4 |
| 10 | 20.130 | 4.40769 | 8.3 |
| 11 | 21.390 | 4.15071 | 4.0 |
| 12 | 22.887 | 3.88257 | 37.3 |
| 13 | 23.793 | 3.73669 | 12.1 |
| 14 | 25.557 | 3.48259 | 24.9 |
| 15 | 25.778 | 3.45332 | 14.4 |
| 16 | 26.263 | 3.39066 | 5.9 |
| 17 | 28.371 | 3.14323 | 3.3 |
| 18 | 30.868 | 2.89444 | 4.1 |

### Example 12: Preparation of Crystal Form F of Compound Represented by Formula (I)

About 8 mg of the compound of formula (I) was taken, and 0.12 mL of N-methylpyrrolidone was added. Crystallization was performed by stirring. The mixture was centrifuged, and the solid was dried *in vacuo* to give a product. According to X-ray powder diffraction analysis, the product was defined as crystal form F. Its XRPD pattern is shown in FIG. 7, and its characteristic peak positions are shown in Table 6. Its DSC thermogram shows endothermic peaks at 96.91 °C, 141.88 °C, and 263.70 °C. Its TGA thermogram shows a weight loss of 25.77% between 30 °C and 220 °C.

**Table 6**

| **Peak No.** | **2θ value [° or degrees]** | **d[Å]** | **Relative intensitv%** |
|---|---|---|---|
| 1 | 6.638 | 13.30424 | 22.6 |
| 2 | 8.775 | 10.06918 | 8.7 |
| 3 | 11.075 | 7.98262 | 20.1 |
| 4 | 12.890 | 6.86227 | 10.2 |
| 5 | 13.610 | 6.50095 | 17.0 |
| 6 | 15.008 | 5.89845 | 38.2 |
| 7 | 16.117 | 5.49482 | 10.8 |
| 8 | 17.676 | 5.01376 | 8.0 |
| 9 | 18.610 | 4.76410 | 10.9 |
| 10 | 18.979 | 4.67220 | 6.3 |
| 11 | 21.151 | 4.19718 | 3.0 |
| 12 | 22.335 | 3.97716 | 17.3 |
| 13 | 22.947 | 3.87253 | 5.4 |
| 14 | 24.247 | 3.66775 | 100.0 |
| 15 | 26.112 | 3.40985 | 11.8 |
| 16 | 27.404 | 3.25199 | 5.7 |
| 17 | 28.164 | 3.16590 | 11.3 |

### Example 13: Preparation of Crystal Form F of Compound Represented by Formula (I)

About 8 mg of the compound of formula (I) was taken, and 0.3 mL of *N*-methylpyrrolidone was added. The compound was dissolved by stirring. 1 mL of methyl *tert*-butyl ether was added, and a solid precipitated. The mixture was centrifuged, and the solid was dried *in vacuo* to give a product. According to X-ray powder diffraction analysis, the product was the crystal form F.

### Example 14: Preparation of Crystal Form G of Compound Represented by Formula (I)

About 8 mg of the compound of formula (I) was taken, and 0.2 mL of dichloromethane was added. The compound was dissolved by stirring. 1.2 mL of isopropyl acetate was added, and a solid precipitated. The mixture was centrifuged, and the solid was dried *in vacuo* to give a product. According to X-ray powder diffraction analysis, the product was defined as crystal form G. Its XRPD pattern is shown in FIG. 8, and its characteristic peak positions are shown in Table 7. Its DSC thermogram shows an endothermic peak at 264.37 °C. Its TGA thermogram shows a weight loss of 0.24% between 30 °C and 205 °C.

**Table 7**

| **Peak No.** | **2θ value [° or degrees]** | **d[Å]** | **Relative intensity%** |
|---|---|---|---|
| 1 | 6.071 | 14.54594 | 100.0 |
| 2 | 11.282 | 7.83666 | 4.9 |
| 3 | 12.253 | 7.21744 | 18.7 |
| 4 | 13.133 | 6.73573 | 6.4 |
| 5 | 14.090 | 6.28057 | 8.3 |
| 6 | 14.203 | 6.23099 | 10.4 |
| 7 | 15.182 | 5.83103 | 5.3 |
| 8 | 16.099 | 5.50101 | 8.3 |
| 9 | 16.938 | 5.23043 | 10.1 |
| 10 | 19.175 | 4.62487 | 17.0 |
| 11 | 21.368 | 4.15490 | 10.4 |
| 12 | 22.402 | 3.96544 | 2.1 |
| 13 | 24.004 | 3.70433 | 14.6 |
| 14 | 26.593 | 3.34926 | 3.4 |
| 15 | 28.813 | 3.09606 | 2.8 |

### Example 15: Preparation of Crystal Form H of Compound Represented by Formula (I)

About 8 mg of the crystal form A of the compound of formula (I) was taken, and 0.8 mL of methanol was added. The mixture was stirred at room temperature, and the compound did not dissolve. The mixture was heated to 50 °C, slowly cooled for precipitation, and centrifuged, and the solid was dried *in vacuo* to give a product. According to X-ray powder diffraction analysis, the product was defined as crystal form H. Its XRPD pattern is shown in FIG. 9, and its characteristic peak positions are shown in Table 8. Its DSC thermogram shows endothermic peaks at 149.99 °C and 264.92 °C and an exothermic peak at 158.39 °C. Its TGA thermogram shows a weight loss of 2.53% between 30 °C and 160 °C.

**Table 8**

| **Peak No.** | **2θ value [° or degrees]** | **d[Å]** | **Relative intensity%** |
|---|---|---|---|
| 1 | 6.841 | 12.90978 | 48.9 |
| 2 | 7.499 | 11.77858 | 100.0 |
| 3 | 9.668 | 9.14111 | 24.5 |
| 4 | 10.191 | 8.67272 | 11.1 |
| 5 | 10.932 | 8.08643 | 14.0 |
| 6 | 12.961 | 6.82481 | 14.1 |
| 7 | 13.980 | 6.32950 | 32.5 |
| 8 | 15.055 | 5.88016 | 32.9 |
| 9 | 16.110 | 5.49719 | 22.4 |
| 10 | 18.017 | 4.91949 | 7.3 |
| 11 | 20.733 | 4.28084 | 3.9 |
| 12 | 21.368 | 4.15492 | 5.4 |
| 13 | 22.119 | 4.01550 | 9.7 |
| 14 | 23.102 | 3.84694 | 10.2 |
| 15 | 25.849 | 3.44395 | 8.8 |
| 16 | 26.482 | 3.36308 | 11.9 |
| 17 | 27.938 | 3.19095 | 5.8 |

### Example 16: Preparation of Crystal Form H of Compound Represented by Formula (I)

About 8 mg of the compound of formula (I) was taken, and 0.25 mL of acetonitrile/methanol (1:1) was added. The compound was dissolved by stirring. 1.2 mL of methyl *tert-*butyl ether was added, and a solid precipitated. The mixture was centrifuged, and the solid was dried *in vacuo* to give a product. According to X-ray powder diffraction analysis, the product was the crystal form H.

### Example 17: Preparation of Crystal Form I of Compound Represented by Formula (I)

About 8 mg of the compound of formula (I) was taken, and 0.4 mL of ethanol was added. The compound was dissolved by stirring. The solution was heated to 50 °C, slowly cooled for crystallization, and centrifuged, and the solid was dried *in vacuo* to give a product. According to X-ray powder diffraction analysis, the product was defined as crystal form I. Its XRPD pattern is shown in FIG. 10, and its characteristic peak positions are shown in Table 9. Its DSC thermogram shows endothermic peaks at 140.16 °C and 264.78 °C and an exothermic peak at 159.54 °C. Its TGA thermogram shows a weight loss of 6.27% between 30 °C and 170 °C.

**Table 9**

| **Peak No.** | **2θ value [° or degrees]** | **d[Å]** | **Relative intensity%** |
|---|---|---|---|
| 1 | 4.857 | 18.17970 | 99.9 |
| 2 | 5.165 | 17.09649 | 100.0 |
| 3 | 7.237 | 12.20549 | 48.3 |
| 4 | 7.921 | 11.15332 | 22.9 |
| 5 | 9.609 | 9.19663 | 57.3 |
| 6 | 9.655 | 9.15286 | 56.8 |
| 7 | 10.223 | 8.64554 | 56.1 |
| 8 | 10.316 | 8.56854 | 53.5 |
| 9 | 10.868 | 8.13401 | 19.6 |
| 10 | 12.009 | 7.36364 | 57.4 |
| 11 | 13.094 | 6.75574 | 41.1 |
| 12 | 13.291 | 6.65629 | 59.7 |
| 13 | 14.507 | 6.10100 | 33.1 |
| 14 | 14.998 | 5.90224 | 38.4 |
| 15 | 15.873 | 5.57876 | 12.2 |
| 16 | 16.441 | 5.38727 | 19.0 |
| 17 | 18.376 | 4.82427 | 14.4 |
| 18 | 19.450 | 4.56007 | 25.8 |
| 19 | 21.154 | 4.19642 | 20.5 |
| 20 | 22.074 | 4.02362 | 55.7 |
| 21 | 23.642 | 3.76028 | 22.1 |
| 22 | 23.881 | 3.72312 | 16.7 |
| 23 | 24.805 | 3.58653 | 7.0 |
| 24 | 25.398 | 3.50402 | 13.9 |
| 25 | 27.114 | 3.28610 | 11.3 |
| 26 | 29.258 | 3.04997 | 6.1 |

### Example 18: Preparation of Crystal Form J of Compound Represented by Formula (I)

About 8 mg of the crystal form A of the compound of formula (I) was taken, and 0.8 mL of isopropanol was added. The mixture was heated to 50 °C, slowly cooled for crystallization, and centrifuged, and the solid was dried *in vacuo* to give a product. According to X-ray powder diffraction analysis, the product was defined as crystal form J. Its XRPD pattern is shown in FIG. 11, and its characteristic peak positions are shown in Table 10. Its DSC thermogram shows endothermic peaks at 153.46 °C and 264.58 °C. Its TGA thermogram shows a weight loss of 9.46% between 30 °C and 160 °C.

**Table 10**

| **Peak No.** | **2θ value [° or degrees]** | **d[Å]** | **Relative intensity%** |
|---|---|---|---|
| 1 | 7.671 | 11.51481 | 100.0 |
| 2 | 10.068 | 8.77893 | 9.0 |
| 3 | 10.687 | 8.27153 | 8.0 |
| 4 | 11.677 | 7.57230 | 24.8 |
| 5 | 13.129 | 6.73789 | 7.1 |
| 6 | 13.480 | 6.56332 | 31.6 |
| 7 | 13.996 | 6.32238 | 34.0 |
| 8 | 14.417 | 6.13870 | 5.5 |
| 9 | 15.465 | 5.72494 | 6.4 |
| 10 | 16.004 | 5.53337 | 7.7 |
| 11 | 16.234 | 5.45549 | 4.9 |
| 12 | 17.260 | 5.13358 | 8.1 |
| 13 | 18.553 | 4.77861 | 41.4 |
| 14 | 20.152 | 4.40280 | 9.9 |
| 15 | 20.742 | 4.27888 | 3.9 |
| 16 | 21.560 | 4.11840 | 11.3 |
| 17 | 21.823 | 4.06931 | 8.5 |
| 18 | 22.881 | 3.88349 | 6.5 |
| 19 | 23.337 | 3.80861 | 10.1 |
| 20 | 24.250 | 3.66735 | 37.4 |
| 21 | 24.813 | 3.58531 | 6.5 |
| 22 | 25.618 | 3.47445 | 5.3 |
| 23 | 26.216 | 3.39653 | 3.3 |
| 24 | 26.354 | 3.37906 | 3.2 |
| 25 | 27.596 | 3.22974 | 4.5 |
| 26 | 28.792 | 3.09824 | 2.0 |
| 27 | 30.011 | 2.97511 | 1.7 |
| 28 | 30.517 | 2.92693 | 3.8 |
| 29 | 32.449 | 2.75693 | 2.2 |

### Example 19: Preparation of Crystal Form K of Compound Represented by Formula (I)

About 8 mg of the crystal form A of the compound of formula (I) was taken, and 0.8 mL of acetonitrile was added. The mixture was heated to 50 °C, slowly cooled for crystallization, and centrifuged, and the solid was dried *in vacuo* to give a product. According to X-ray powder diffraction analysis, the product was defined as crystal form K. Its XRPD pattern is shown in FIG. 12, and its characteristic peak positions are shown in Table 11. Its DSC thermogram shows endothermic peaks at 153.65 °C, 160.48 °C, and 265.14 °C and an exothermic peak at 158.85 °C. Its TGA thermogram shows a weight loss of 5.27% between 30 °C and 195 °C.

**Table 11**

| **Peak No.** | **2θ value [° or degrees]** | **d[Å]** | **Relative intensity%** |
|---|---|---|---|
| 1 | 6.800 | 12.98903 | 80.2 |
| 2 | 7.449 | 11.85870 | 100.0 |
| 3 | 9.504 | 9.29808 | 25.0 |
| 4 | 10.942 | 8.07927 | 8.3 |
| 5 | 12.831 | 6.89379 | 8.1 |
| 6 | 13.783 | 6.41960 | 26.7 |
| 7 | 14.187 | 6.23780 | 26.6 |
| 8 | 14.773 | 5.99156 | 11.3 |
| 9 | 14.995 | 5.90343 | 29.2 |
| 10 | 15.766 | 5.61647 | 37.2 |
| 11 | 16.133 | 5.48955 | 14.0 |
| 12 | 17.619 | 5.02985 | 9.9 |
| 13 | 17.934 | 4.94216 | 12.4 |
| 14 | 18.417 | 4.81342 | 9.7 |
| 15 | 19.212 | 4.61604 | 2.9 |
| 16 | 20.050 | 4.42513 | 4.0 |
| 17 | 20.670 | 4.29378 | 28.3 |
| 18 | 21.229 | 4.18183 | 4.9 |
| 19 | 22.102 | 4.01862 | 42.2 |
| 20 | 22.337 | 3.97692 | 14.1 |
| 21 | 22.613 | 3.92887 | 8.0 |
| 22 | 23.918 | 3.71739 | 5.5 |
| 23 | 24.625 | 3.61235 | 6.7 |
| 24 | 25.807 | 3.44950 | 9.8 |
| 25 | 26.145 | 3.40569 | 10.5 |
| 26 | 26.548 | 3.35480 | 13.4 |
| 27 | 27.635 | 3.22526 | 18.2 |
| 28 | 29.680 | 3.00758 | 7.3 |
| 29 | 30.918 | 2.88994 | 3.1 |
| 30 | 33.016 | 2.71088 | 2.8 |

### Example 20: Preparation of Crystal Form L of Compound Represented by Formula (I)

About 200 mg of the compound of formula (I) was taken, and 5 mL of dichloromethane was added. The compound was dissolved by stirring. 30 mL of methyl *tert*-butyl ether was added, and a solid precipitated. The mixture was centrifuged, and the solid was dried *in vacuo* to give a product. According to X-ray powder diffraction analysis, the product was defined as crystal form L. Its XRPD pattern is shown in FIG. 13, and its characteristic peak positions are shown in Table 12. Its DSC thermogram shows endothermic peaks at 137.47 °C and 264.18 °C and an exothermic peak at 159.83 °C. Its TGA thermogram shows a weight loss of 9.46% between 30 °C and 195 °C.

**Table 12**

| **Peak No.** | **2θ value [° or degrees]** | **d[Å]** | **Relative intensity%** |
|---|---|---|---|
| 1 | 4.946 | 17.85130 | 13.6 |
| 2 | 5.540 | 15.93928 | 7.8 |
| 3 | 6.185 | 14.27750 | 6.5 |
| 4 | 7.118 | 12.40843 | 38.2 |
| 5 | 7.522 | 11.74254 | 30.2 |
| 6 | 7.771 | 11.36721 | 16.4 |
| 7 | 8.014 | 11.02330 | 11.3 |
| 8 | 9.202 | 9.60320 | 10.3 |
| 9 | 9.450 | 9.35098 | 10.4 |
| 10 | 10.663 | 8.29008 | 7.9 |
| 11 | 12.311 | 7.18379 | 15.8 |
| 12 | 12.756 | 6.93407 | 100.0 |
| 13 | 13.586 | 6.51241 | 14.3 |
| 14 | 13.959 | 6.33916 | 13.0 |
| 15 | 14.114 | 6.26968 | 13.2 |
| 16 | 14.923 | 5.93179 | 4.9 |
| 17 | 15.265 | 5.79964 | 4.8 |
| 18 | 16.105 | 5.49914 | 7.9 |
| 19 | 18.841 | 4.70620 | 10.4 |
| 20 | 19.494 | 4.55001 | 19.1 |
| 21 | 19.899 | 4.45822 | 11.1 |
| 22 | 21.080 | 4.21116 | 10.4 |
| 23 | 22.821 | 3.89362 | 2.9 |
| 24 | 23.692 | 3.75247 | 3.9 |
| 25 | 24.562 | 3.62140 | 10.1 |
| 26 | 25.029 | 3.55496 | 2.5 |
| 27 | 25.836 | 3.44565 | 25.4 |
| 28 | 27.609 | 3.22823 | 1.9 |
| 29 | 28.667 | 3.11154 | 8.3 |
| 30 | 29.819 | 2.99388 | 2.9 |

### Example 21: Preparation of Crystal Form M of Compound Represented by Formula (I)

About 8 mg of the compound of formula (I) was taken, and 0.48 mL of dimethyl sulfoxide was added. The compound was dissolved by stirring, and crystallization was performed by volatilization at room temperature to give a product. According to X-ray powder diffraction analysis, the product was defined as crystal form M. Its XRPD pattern is shown in FIG. 14, and its characteristic peak positions are shown in Table 13. Its DSC thermogram shows endothermic peaks at 161.65 °C and 264.86 °C. Its TGA thermogram shows a weight loss of 13.41% between 30 °C and 235 °C.

**Table 13**

| **Peak No.** | **2θ value [° or degrees]** | **d[Å]** | **Relative intensity%** |
|---|---|---|---|
| 1 | 6.618 | 13.34515 | 55.6 |
| 2 | 7.135 | 12.37965 | 17.5 |
| 3 | 7.684 | 11.49577 | 6.6 |
| 4 | 8.595 | 10.27894 | 9.1 |
| 5 | 9.244 | 9.55902 | 17.9 |
| 6 | 10.889 | 8.11851 | 21.5 |
| 7 | 11.471 | 7.70795 | 11.9 |
| 8 | 12.848 | 6.88496 | 46.2 |
| 9 | 13.240 | 6.68196 | 13.4 |
| 10 | 13.924 | 6.35492 | 8.6 |
| 11 | 14.485 | 6.11017 | 17.8 |
| 12 | 14.770 | 5.99301 | 66.9 |
| 13 | 15.337 | 5.77248 | 35.1 |
| 14 | 15.893 | 5.57199 | 14.9 |
| 15 | 16.292 | 5.43629 | 11.7 |
| 16 | 17.119 | 5.17540 | 10.2 |
| 17 | 17.604 | 5.03391 | 6.0 |
| 18 | 18.004 | 4.92313 | 18.4 |
| 19 | 19.173 | 4.62537 | 7.6 |
| 20 | 19.949 | 4.44711 | 100.0 |
| 21 | 20.514 | 4.32598 | 4.3 |
| 22 | 21.027 | 4.22149 | 12.6 |
| 23 | 21.370 | 4.15463 | 6.2 |
| 24 | 21.997 | 4.03750 | 14.9 |
| 25 | 22.511 | 3.94655 | 2.3 |
| 26 | 23.081 | 3.85027 | 12.0 |
| 27 | 23.823 | 3.73205 | 3.1 |
| 28 | 24.479 | 3.63348 | 13.9 |
| 29 | 24.888 | 3.57470 | 27.9 |
| 30 | 25.333 | 3.51295 | 43.1 |
| 31 | 25.906 | 3.43657 | 42.8 |
| 32 | 26.039 | 3.41922 | 40.0 |
| 33 | 26.796 | 3.32440 | 6.7 |
| 34 | 26.994 | 3.30042 | 23.8 |
| 35 | 28.083 | 3.17486 | 83.4 |
| 36 | 28.730 | 3.10485 | 6.3 |
| 37 | 29.072 | 3.06906 | 14.6 |
| 38 | 29.945 | 2.98152 | 56.7 |
| 39 | 31.468 | 2.84061 | 3.0 |
| 40 | 31.925 | 2.80103 | 3.5 |
| 41 | 32.324 | 2.76733 | 7.3 |
| 42 | 33.408 | 2.67997 | 4.8 |
| 43 | 35.034 | 2.55922 | 2.5 |
| 44 | 35.776 | 2.50784 | 9.5 |

### Example 22: Preparation of Crystal Form N of Compound Represented by Formula (I)

10 mg of the compound represented by formula (I) was taken, and 0.5 mL of isopropanol was added. The mixture was stirred at 50 °C to completely dissolve the compound, and 11 µL of a 2 M aqueous phosphoric acid solution was added. The mixture was cooled for crystallization and centrifuged, and the solid was dried *in vacuo* to give a product. According to X-ray powder diffraction analysis, the product was the crystal form N. Its XRPD pattern is shown in FIG. 15, and its characteristic peak positions are shown in Table 14. Its DSC thermogram shows an endothermic peak at 226.58 °C. Its TGA thermogram shows a weight loss of 4.51% between 30 °C and 235 °C.

**Table 14**

| **Peak No.** | **2θ value [° or degrees]** | **d[Å]** | **Relative intensity%** |
|---|---|---|---|
| 1 | 5.823 | 15.16583 | 100.0 |
| 2 | 7.608 | 11.61132 | 74.4 |
| 3 | 10.982 | 8.04984 | 10.9 |
| 4 | 13.548 | 6.53046 | 11.9 |
| 5 | 14.888 | 5.94544 | 3.2 |
| 6 | 15.486 | 5.71732 | 4.8 |
| 7 | 16.009 | 5.53158 | 23.7 |
| 8 | 16.246 | 5.45148 | 25.0 |
| 9 | 16.368 | 5.41125 | 14.6 |
| 10 | 16.680 | 5.31081 | 7.1 |
| 10 | 17.901 | 4.95124 | 15.8 |
| 11 | 18.641 | 4.75631 | 25.3 |
| 12 | 19.779 | 4.48497 | 6.2 |
| 13 | 20.334 | 4.36378 | 9.9 |
| 14 | 21.204 | 4.18683 | 9.4 |
| 15 | 22.037 | 4.03033 | 18.8 |
| 16 | 22.297 | 3.98396 | 17.1 |
| 17 | 23.030 | 3.85873 | 8.8 |
| 18 | 24.048 | 3.69763 | 11.9 |
| 19 | 24.508 | 3.62922 | 5.1 |
| 20 | 25.241 | 3.52554 | 4.2 |
| 21 | 25.347 | 3.51100 | 6.4 |
| 22 | 26.577 | 3.35123 | 9.0 |
| 23 | 27.373 | 3.25556 | 5.4 |
| 24 | 27.540 | 3.23622 | 3.6 |
| 25 | 27.984 | 3.18584 | 3.0 |
| 26 | 29.464 | 3.02908 | 5.8 |
| 27 | 29.863 | 2.98952 | 3.8 |

### Example 23: Hygroscopicity Study of Crystal Form D of Compound of Formula (I)

The study was conducted using the Surface Measurement Systems advantage 2 at 25°C, starting from a humidity of 50%, covering a humidity range of 0% to 95% in 10% increments. The criterion for judgment was that the mass change dM/dT is less than 0.002% for each gradient. The running time TMAX was 360 min for each humidity gradient. The study contained two cycles.

**Table 15**

| Test sample | 0.0%RH-95.0%RH | 0%RH-80.0%RH | Crystal form |
|---|---|---|---|
| Crystal form D | 1.74% | 1.40% (slightly hygroscopic) | Did not change |

### Example 24: Influencing Factor Experiments of Crystal Forms of Compound of Formula (I)

The crystal forms D, E, G, H, and N of the compound of formula (I) were spread out in open containers and left to stand under conditions of high temperatures (40 °C and 60 °C) and high humidities (RH 75% and RH 92.5%) for 30 days to investigate their stability.

**Table 16**

| Conditions | Time (days) | Free-state crystal form D | | |
|---|---|---|---|---|
| | | Color and state | Purity% | Crystal form |
| Initial | 0 | Off-white solid | 97.6 | Crystal form D |
| | 6 | Off-white solid | 97.6 | Did not change |
| 40 °C | 14 | Off-white solid | 97.5 | Did not change |
| | 30 | Off-white solid | 97.4 | Did not change |
| | 6 | Off-white solid | 97.5 | Did not change |
| 60 °C | 14 | Off-white solid | 97.5 | Did not change |
| | 30 | Off-white solid | 97.3 | Did not change |
| 75% RH | 6 | Off-white solid | 97.6 | Did not change |
| | 14 | Off-white solid | 97.6 | Did not change |
| | 30 | Off-white solid | 97.6 | Did not change |
| | 6 | Off-white solid | 97.6 | Did not change |
| 92.5% RH | 14 | Off-white solid | 97.6 | Did not change |
| | 30 | Off-white solid | 97.6 | Did not change |

| Conditions | Time (days) | Free-state crystal form E | | |
|---|---|---|---|---|
| | | Color and state | Purity% | Crystal form |
| Initial | 0 | Off-white solid | 98.9 | Crystal form E |
| | 6 | Off-white solid | 98.9 | Did not change |
| 40 °C | 14 | Off-white solid | 98.9 | Did not change |
| | 30 | Off-white solid | 98.9 | Did not change |
| | 6 | Off-white solid | 98.9 | Did not change |
| 60 °C | 14 | Off-white solid | 98.9 | Did not change |
| | 30 | Off-white solid | 98.8 | Did not change |
| | 6 | Off-white solid | 98.9 | Partially changed |
| 75% RH | 14 | Off-white solid | 98.9 | Partially changed |
| | 30 | Off-white solid | 98.9 | Partially changed |
| | 6 | Off-white solid | 98.9 | Changed |
| 92.5% RH | 14 | Off-white solid | 99.0 | Changed |
| | 30 | Off-white solid | 98.9 | Changed |

| Conditions | Time (days) | Free-state crystal form G | | |
|---|---|---|---|---|
| | | Color and state | Purity% | Crystal form |
| Initial | 0 | Off-white solid | 98.6 | Crystal form G |
| | 8 | Off-white solid | 98.6 | Did not change |
| 40 °C | 14 | Off-white solid | 98.4 | Did not change |
| | 30 | Off-white solid | 98.1 | Did not change |
| | 8 | Off-white solid | 98.5 | Did not change |
| 60 °C | 14 | Off-white solid | 98.3 | Did not change |
| | 30 | Off-white solid | 98.0 | Did not change |
| | 8 | Off-white solid | 98.7 | Changed |
| 75% RH | 14 | Off-white solid | 98.7 | Changed |
| | 30 | Off-white solid | 98.7 | Changed |
| | 8 | Off-white solid | 98.7 | Changed |
| 92.5% RH | 14 | Off-white solid | 98.7 | Changed |
| | 30 | Off-white solid | 98.7 | Changed |

| Conditions | Time (days) | Free-state crystal form H | | |
|---|---|---|---|---|
| | | Color and state | Purity% | Crystal form |
| Initial | 0 | Off-white solid | 99.4 | Crystal form H |
| | 7 | Off-white solid | 99.1 | Did not change |
| 40 °C | 14 | Off-white solid | 99.1 | Did not change |
| | 30 | Off-white solid | 99.0 | Did not change |
| | 7 | Off-white solid | 98.9 | Did not change |
| 60 °C | 14 | Off-white solid | 98.7 | Did not change |
| | 30 | Off-white solid | 98.2 | Did not change |
| | 7 | Off-white solid | 99.3 | Did not change |
| 75% RH | 14 | Off-white solid | 99.3 | Partially changed |
| | 30 | Off-white solid | 99.3 | Partially changed |
| | 7 | Off-white solid | 99.2 | Changed |
| 92.5% RH | 14 | Off-white solid | 99.3 | Changed |
| | 30 | Off-white solid | 99.3 | Changed |

Conclusion: The influencing factor experiments indicate that standing under conditions of high temperatures (40 °C and 60 °C) and high humidities (75% and 92.5%) for 30 days, the crystal form D of the compound of formula (I) exhibited good physical and chemical stability; standing under conditions of high humidities (75% and 92.5%) for 30 days, the crystal forms E, G, and H exhibited poor physical stability and good chemical stability.

### Example 25: Long-Term/Accelerated Stability of Crystal Forms of Compound of Formula (I)

The crystal forms D, E, G, and H of the compound of formula (I) were left to stand under conditions of -20 °C, 4 °C, and 25 °C/60% RH and 40 °C/75% RH to investigate their stability.

| Table 17 sample | Standing conditions | Purity% | Purity% | Purity% | Purity% | Purity% | Crystal form |
|---|---|---|---|---|---|---|---|
| | | Initial | 1 month | 2 months | 3 months | 6 months | |
| Crystal form D | 25 °C/60% RH | 97.6 | 97.7 | 97.7 | 97.6 | 97.6 | D |
| | 40 °C/75% RH | 97.6 | 97.7 | 97.6 | 97.6 | 97.6 | D |

| Sample | Standing conditions | Purity% | Purity% | Purity% | Purity% | Purity% | Crystal form |
|---|---|---|---|---|---|---|---|
| | | Initial | 1 month | 2 months | 3 months | 6 months | |
| Crystal form E | 25 °C/60% RH | 98.9 | 98.9 | 98.9 | 98.9 | 98.9 | E |
| | 40 °C/75% RH | 98.9 | 98.9 | 98.9 | 98.9 | 98.9 | Partially changed |

| Sample | Standing conditions | Purity% Purity% | Purity% Purity% | Purity% Purity% | Purity% Purity% | Purity% | Crystal form |
|---|---|---|---|---|---|---|---|
| | | Initial | 1 month | 2 months | 3 months | 6 months | |
| Crystal form G | 25 °C/60% RH | 98.6 | 98.7 | 98.7 | 98.7 | 98.7 | G |
| | 40 °C/75% RH | 98.6 | 98.7 | 98.7 | 98.7 | 98.7 | Changed |

| Sample | Standing | Purity% | Purity% | Purity% | Purity% | Purity% | Crystal form |
|---|---|---|---|---|---|---|---|
| | conditions | Initial | 14 days | 2 months | 3 months | 6 months | |
| Crystal form H | 25 °C/60% RH | 99.4 | 99.3 | 99.3 | 99.3 | 99.4 | H |
| | 40 °C/75% RH | 99.4 | 99.3 | 99.3 | 99.3 | 99.3 | Changed |

Conclusion: The long-term accelerated experiments indicate that standing under conditions of 25 °C/60RH and 40 °C/75RH for 6 months, the crystal form D exhibited good physical and chemical stability, and the crystal forms E, G, and H exhibited good chemical stability and slightly inferior physical stability.

## Claims

1. A crystal form B of the compound represented by formula (I): 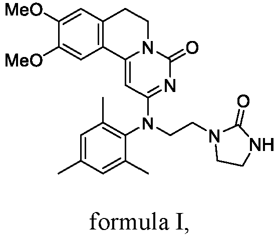 wherein an X-ray powder diffraction pattern expressed in terms of 2*θ* angles, which are diffraction angles, of the crystal form has characteristic peaks at 6.6, 11.0, 12.7, 15.5, 19.1, 23.7, and 25.8, preferably 6.6, 10.0, 11.0, 11.7, 12.7, 15.5, 19.1, 23.7, 24.6, and 25.8, and more preferably 6.6, 8.6, 10.0, 11.0, 11.7, 12.7, 15.5, 17.8, 19.1, 23.7, 24.6, 25.8, and 26.9; most preferably, the X-ray powder diffraction pattern expressed in terms of 2θ angles, which are diffraction angles, is shown in FIG. 3.

2. A crystal form C of the compound represented by formula (I): 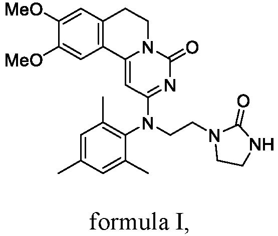 wherein an X-ray powder diffraction pattern expressed in terms of 2*θ* angles, which are diffraction angles, of the crystal form has characteristic peaks at 6.6, 7.5, 10.1, 10.9, 15.6, 23.8, and 24.3, preferably 6.6, 7.5, 10.1, 10.9, 13.8, 15.6, 20.7, 23.8, 24.3, and 24.7, and more preferably 6.6, 7.5, 10.1, 10.9, 13.8, 15.6, 17.5, 19.3, 20.7, 23.8, 24.3, 24.7, and 27.0; most preferably, the X-ray powder diffraction pattern expressed in terms of 2*θ* angles, which are diffraction angles, is shown in FIG. 4.

3. A crystal form D of the compound represented by formula (I): 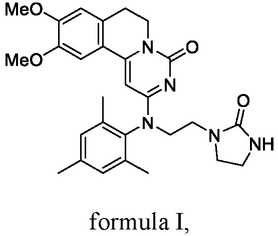 wherein an X-ray powder diffraction pattern expressed in terms of 2*θ* angles, which are diffraction angles, of the crystal form has characteristic peaks at 6.6, 10.1, 11.0, 15.6, 16.5, 17.5, and 24.2, preferably 6.6, 10.1, 11.0, 15.6, 16.5, 17.5, 20.3, 24.2, 25.8, and 26.9, and more preferably 6.6, 10.1, 11.0, 15.6, 16.5, 17.5, 20.3, 23.3, 24.2, 24.7, 25.1, 25.8, and 26.9; most preferably, the X-ray powder diffraction pattern expressed in terms of 2*θ* angles, which are diffraction angles, is shown in FIG. 5.

4. A crystal form E of the compound represented by formula (I): 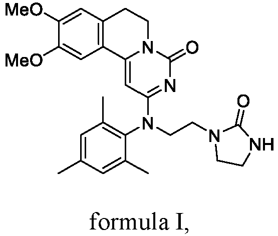 wherein an X-ray powder diffraction pattern expressed in terms of 2*θ* angles, which are diffraction angles, of the crystal form has characteristic peaks at 6.3, 11.1, 12.8, 14.3, 17.9, 22.9, and 25.6, preferably 6.3, 11.1, 12.8, 14.3, 17.0, 17.9, 18.7, 22.9, 23.8, and 25.6, and more preferably 6.3, 11.1, 12.8, 14.3, 15.9, 17.0, 17.9, 18.7, 20.1, 22.9, 23.8, and 25.6; most preferably, the X-ray powder diffraction pattern expressed in terms of 2*θ* angles, which are diffraction angles, is shown in FIG. 6.

5. A crystal form F of the compound represented by formula (I): 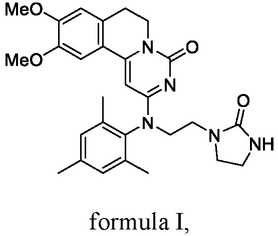 wherein an X-ray powder diffraction pattern expressed in terms of 2*θ* angles, which are diffraction angles, of the crystal form has characteristic peaks at 6.6, 11.1, 13.6, 15.0, 22.3, 24.2, and 26.1, preferably 6.6, 11.1, 13.6, 15.0, 16.1, 18.6, 22.3, 24.2, 26.1, and 28.1, and more preferably 6.6, 8.8, 11.1, 12.9, 13.6, 15.0, 16.1, 17.7, 18.6, 22.3, 24.2, 26.1, and 28.2; most preferably, the X-ray powder diffraction pattern expressed in terms of 2*θ* angles, which are diffraction angles, is shown in FIG. 7.

6. A crystal form G of the compound represented by formula (I): 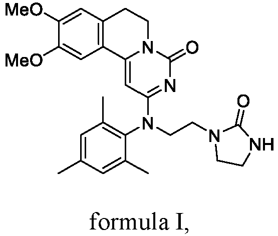 wherein an X-ray powder diffraction pattern expressed in terms of 2*θ* angles, which are diffraction angles, of the crystal form has characteristic peaks at 6.1, 12.3, 14.2, 16.9, 19.2, 21.4, and 24.0, preferably 6.1, 12.3, 13.1, 14.2, 15.2, 16.1, 16.9, 19.2, 21.4, and 24.0, and more preferably 6.1, 11.3, 12.3, 13.1, 14.2, 15.2, 16.1, 16.9, 19.2, 21.4, 24.0, 26.6, and 28.8; most preferably, the X-ray powder diffraction pattern expressed in terms of 2*θ* angles, which are diffraction angles, is shown in FIG. 8.

7. A crystal form H of the compound represented by formula (I): 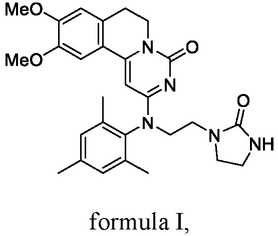 wherein an X-ray powder diffraction pattern expressed in terms of 2*θ* angles, which are diffraction angles, of the crystal form has characteristic peaks at 6.8, 7.5, 9.7, 13.0, 14.0, 15.1, and 16.1, preferably 6.8, 7.5, 9.7, 10.2, 10.9, 13.0, 14.0, 15.1, 16.1, and 26.5, and more preferably 6.8, 7.5, 9.7, 10.2, 10.9, 13.0, 14.0, 15.1, 16.1, 22.1, 23.1, 25.8, and 26.5; most preferably, the X-ray powder diffraction pattern expressed in terms of 2θ angles, which are diffraction angles, is shown in FIG. 9.

8. A crystal form I of the compound represented by formula (I): 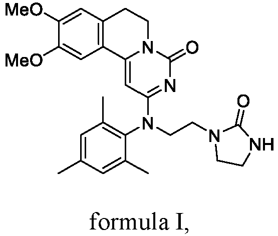 wherein an X-ray powder diffraction pattern expressed in terms of 2*θ* angles, which are diffraction angles, of the crystal form has characteristic peaks at 5.2, 7.2, 9.6, 10.2, 12.0, 13.3, and 22.1, preferably 5.2, 7.2, 9.6, 10.2, 12.0, 13.3, 14.5, 15.0, 19.5, and 22.1, and more preferably 5.2, 7.2, 7.9, 9.6, 10.2, 12.0, 13.3, 14.5, 15.0, 19.5, 21.2, 22.1, and 23.6; most preferably, the X-ray powder diffraction pattern expressed in terms of 2θ angles, which are diffraction angles, is shown in FIG. 10.

9. A crystal form J of the compound represented by formula (I): 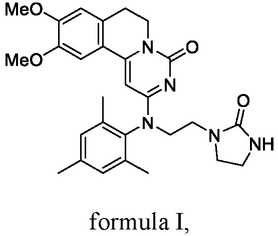 wherein an X-ray powder diffraction pattern expressed in terms of 2*θ* angles, which are diffraction angles, of the crystal form has characteristic peaks at 7.7, 11.7, 13.5, 14.0, 18.6, 21.6, and 24.3, preferably 7.7, 10.1, 11.7, 13.5, 14.0, 18.6, 20.2, 21.6, 23.3, and 24.3, and more preferably 7.7, 10.1, 10.7, 11.7, 13.5, 14.0, 16.0, 17.3, 18.6, 20.2, 21.6, 23.3, and 24.3; most preferably, the X-ray powder diffraction pattern expressed in terms of 2θ angles, which are diffraction angles, is shown in FIG. 11.

10. A crystal form K of the compound represented by formula (I): 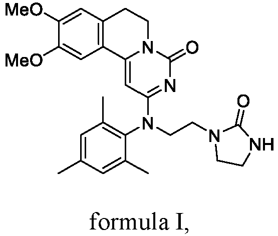 wherein an X-ray powder diffraction pattern expressed in terms of 2*θ* angles, which are diffraction angles, of the crystal form has characteristic peaks at 6.8, 7.4, 13.8, 15.0, 15.8, 20.7, and 22.1, preferably 6.8, 7.4, 9.5, 13.8, 14.2, 15.0, 15.8, 20.7, 22.1, and 27.6, and more preferably 6.8, 7.4, 9.5, 13.8, 14.2, 15.0, 15.8, 17.9, 18.4, 20.7, 22.1, 26.5, and 27.6; most preferably, the X-ray powder diffraction pattern expressed in terms of 2θ angles, which are diffraction angles, is shown in FIG. 12.

11. A crystal form L of the compound represented by formula (I): 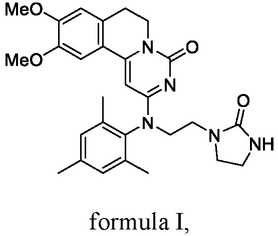 wherein an X-ray powder diffraction pattern expressed in terms of 2*θ* angles, which are diffraction angles, of the crystal form has characteristic peaks at 7.1, 7.6, 12.8, 13.6, 19.5, 21.1, and 25.8, preferably 7.1, 7.6, 9.2, 12.8, 13.6, 16.1, 19.5, 21.1, 24.6, and 25.8, and more preferably 7.1, 7.6, 9.2, 12.8, 13.6, 16.1, 19.5, 21.1, 24.6, and 25.8; most preferably, the X-ray powder diffraction pattern expressed in terms of 2θ angles, which are diffraction angles, is shown in FIG. 13.

12. A crystal form M of the compound represented by formula (I): 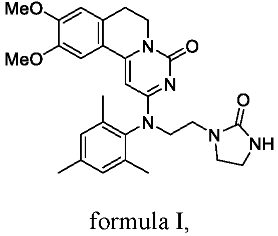 wherein an X-ray powder diffraction pattern expressed in terms of 2*θ* angles, which are diffraction angles, of the crystal form has characteristic peaks at 6.6, 12.8, 14.8, 19.9, 25.3, 28.1, and 29.9, preferably 6.6, 12.8, 14.8, 15.3, 19.9, 24.9, 25.3, 25.9, 28.1, and 29.9, and more preferably 6.6, 10.9, 12.8, 14.8, 15.3, 18.0, 19.9, 24.9, 25.3, 25.9, 27.0, 28.1, and 29.9; most preferably, the X-ray powder diffraction pattern expressed in terms of 2θ angles, which are diffraction angles, is shown in FIG. 14.

13. A crystal form N of the compound represented by formula (I): 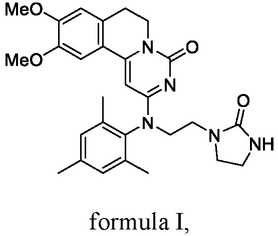 wherein an X-ray powder diffraction pattern expressed in terms of 2*θ* angles, which are diffraction angles, of the crystal form has characteristic peaks at 5.8, 7.6, 16.0, 16.2, 18.6, 22.0, and 22.3, preferably 5.8, 7.6, 13.5, 16.0, 16.2, 17.9, 18.6, 22.0, 22.3, and 24.0, and more preferably 5.8, 7.6, 11.0, 13.5, 16.0, 16.2, 17.9, 18.6, 20.3, 21.2, 22.0, 22.3, and 24.0; most preferably, the X-ray powder diffraction pattern expressed in terms of 2θ angles, which are diffraction angles, is shown in FIG. 15.

14. The crystal form according to any one of claims 1-13, wherein the 2*θ* angles have a margin of error of ±0.2.

15. A pharmaceutical composition prepared from the crystal form according to any one of claims 1-14.

16. A pharmaceutical composition, comprising the crystal form according to any one of claims 1-14 and optionally a pharmaceutically acceptable carrier, diluent, or excipient.

17. A preparation method for a pharmaceutical composition, comprising a step of mixing the crystal form according to any one of claims 1-14 with a pharmaceutically acceptable carrier, diluent, or excipient.

18. Use of the crystal form according to any one of claims 1-14, or the pharmaceutical composition according to claim 15 or 16, or a pharmaceutical composition prepared by the method according to claim 17 in preparing a medicament for preventing and/or treating a PDE-related disorder.

19. Use of the crystal form according to any one of claims 1-14, or the pharmaceutical composition according to claim 15 or 16, or a pharmaceutical composition prepared by the method according to claim 17 in preparing a medicament for preventing and/or treating asthma, obstructive pulmonary disease, sepsis, nephritis, diabetes, allergic rhinitis, allergic conjunctivitis, ulcerative enteritis, or rheumatism.
